# EUROPÄISCHE PATENTSCHRIFT

(11) **EP 0 503 561 B1**
(45) Veröffentlichungstag und Bekanntmachung des Hinweises auf die Patenterteilung: **28.10.1998**
(21) Anmeldenummer: 92104085.3
(22) Anmeldetag: 10.03.1992
(51) Int. Cl.: C07C 269/06, C07C 315/04, C07D 217/16, C07C 271/20, C07C 271/22, C07C 317/44

(54) **Verfahren zur diastereoselektiven reduktiven Pinakol-Kupplung von homochiralen alpha-Aminoaldehyden**
Method for the diastereoselective reductive pinacol coupling of homochiral alpha aminoaldehydes
Procédé pour l'accouplement diastereoselective reductif des alpha aminoaldehydes homochirales

(30) Priorität: 15.03.1991 DE 4108357; 11.07.1991 DE 4122911
(43) Veröffentlichungstag der Anmeldung: 16.09.1992
(73) Patentinhaber: HOECHST AKTIENGESELLSCHAFT, 65926 Frankfurt am Main (DE)
(72) Erfinder: Jendralla, Joachim-Heiner, Dr., W-6230 Frankfurt am Main 80 (DE); Jacobi, Detlef, Dr., W-6000 Frankfurt am Main (DE); Kammermeier, Bernhard, Dr., W-6000 Frankfurt am Main 71 (DE)

(56) Entgegenhaltungen:
- EP-A- 0 402 646
- WO-A-91/18866
- JOURNAL OF ORGANIC CHEMISTRY Bd. 57, Nr. 1, Januar 1992, WASHINGTON,D.C. Seiten 28 - 32 PEDERSEN S.F. ET AL 'Pinacol homocoupling of (S)-2-(n-(benzyloxycarbonyl)amino) aldehydes by(V2Cl3(THF)6)2(Zn2Cl6).'
- JOURNAL OF MEDICINAL CHEMISTRY Bd. 33, Nr. 10, 1990, WASHINGTON,D.C. Seiten 2687 - 2689 KEMPF D.F. ET AL 'Structure-based,C2 symmetric inhibitors of HIV protease'
- JOURNAL OF ORGANIC CHEMISTRY Bd. 55, 1990, WASHINGTON,D.C. Seiten 4506 - 4508 PEDERSEN S.F. ET AL 'Stereoselective synthesis of 3-amino 1,2-diols via intermolecular pinacol cross-coupling of alpha-((alkoxycarbonyl)amino)aldehydes with aliphatic aldehydes.'
- TETRAHEDRON LETTERS Bd. 30, Nr. 51, 1989, OXFORD,G.B. Seiten 7177 - 7180 PEDERSEN S.F. ET AL 'Intermolecular pinacol cross coupling of aryl aldehydes or their dimethyl acetals with non-aryl aldehydes'
- JOURNAL OF THE AMERICAN CHEMICAL SOCIETY Bd. 111, 1989, WASHINGTON,D.C. Seiten 8014 - 8016 PEDERSEN S.F. ET AL 'Intermolecular pinacol cross coupling of electronically similar aldehydes.'

## Beschreibung

Die Erfindung betrifft ein Verfahren zur Herstellung von optisch reinen symmetrischen Verbindungen der Formel I 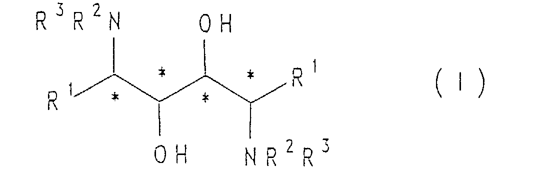 worin R¹, R² und R³ unten näher erläutert wird, unter gleichzeitiger Kontrolle der vier mit * gekennzeichneten Chiralitätszentren.

In EP-A 0402646 und D.J.Kempf et al. [J.Med.Chem. 33, 2687 (1990)] ist die Herstellung von Verbindungen obigen Typs unter Verwendung von McMurry-Reagenz [TiCl₃/Zn(Cu)] beschrieben. Diese reduktive Kupplungsmethode führt zu schwer trennbaren Gemischen. Wie in den oben zitierten Arbeiten berichtet, entstehen die drei Diastereomeren in etwa gleicher Menge und schlechter Ausbeute.

S.F.Pedersen et al. [J.Am.Chem.Soc. 111, 8014 (1989)] beschreiben, daß sich mit dem Vanadium(II)-Komplex [V₂Cl₃(THF)₆]₂[Zn₂Cl₆] bei der reduktiven Kreuzkupplung von achiralen linearen aliphatischen Aldehyden mit 3-Formyl-propanamiden syn-Diole diastereoselektiv erzielen lassen, wobei durch Einsatz unterschiedlicher Carbonylverbindungen konstitutionell unsymmetrische Verbindungen entstehen.

Der Einsatz des gleichen Vanadium (II)-Komplexes zür redüktiven Küpplung von Aldehyden wird in dem Dokument WO 91/18866 (Veröffentlichungstag 12.12.91) beschrieben.

Ziel der vorliegenden Erfindung ist es, ein einfacheres und stereoselektives Verfahren zur Herstellung der obengenannten Verbindungen zu finden, welches die bekannten Nachteile nicht besitzt.

Die Erfindung wird gelöst durch das Verfahren zur Herstellung von Verbindungen der Formel I 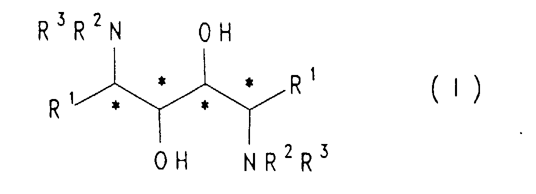 worin
- R¹: für eine Seitenkettenrest einer natürlichen oder nicht-natürlichen α-Aminosäure steht;
- R² und R³: gleich oder verschieden sind und

a)
   - Wasserstoff
b)
   - einen Rest der Formel

      D-(E)ₙ-(F)ₒ-(G)ₚ (II)

      bedeuten, wobei E, F und G unabhängig voneinander für eine natürliche oder unnatürliche Aminosäure, Azaaminosäure oder Iminosäure stehen;
      n, o, p unabhängig voneinander 0 oder 1 bedeuten;
      D für R⁴ oder einen Rest der Formeln III, IV oder V steht 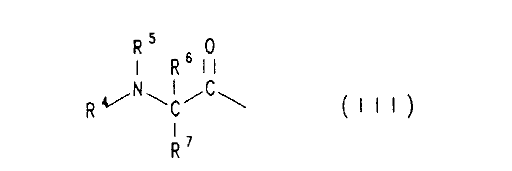 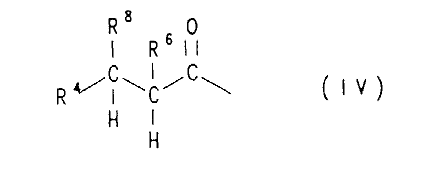 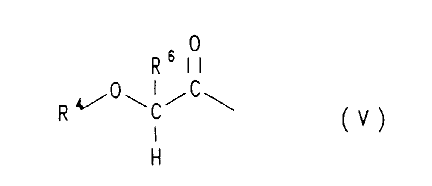 worin R⁴ bedeutet
b₁)
   - Wasserstoff,
   - Carboxyl,
   - (C₁-C₁₈)-Alkyl, das gegebenenfalls einfach oder zweifach ungesättigt ist und das gegebenenfalls durch bis zu 3 gleiche oder verschiedene Reste aus der Reihe
      - Mercapto,
      - Hydroxy,
      - (C₁-C₇)-Alkoxy,
      - Carbamoyl,
      - (C₁-C₈)-Alkanoyloxy,
      - Carboxy,
      - (C₁-C₇)-Alkoxycarbonyl,
      - F, Cl, Br, I,
      - Amino,
      - Amidino, das gegebenenfalls durch einen, zwei oder drei (C₁-C₈)-Alkylreste substituiert sein kann,
      - Guanidino, das gegebenenfalls durch einen oder zwei Benzyloxycarbonylreste oder durch einen, zwei, drei oder vier (C₁-C₈)-Alkylreste substituiert sein kann,
      - (C₁-C₇)-Alkylamino,
      - Di-(C₁-C₇)-Alkylamino,
      - (C₁-C₆)-Alkoxycarbonylamino,
      - (C₇-C₁₅)-Aralkoxycarbonyl,
      - (C₇-C₁₅)-Aralkoxycarbonylamino,
      - Phenyl-(C₁-C₄)-alkoxy,
      - 9-Fluorenylmethoxycarbonylamino,
      - (C₁-C₆)-Alkylsulfonyl,
      - (C₁-C₆)-Alkylsulfinyl,
      - (C₁-C₆)-Alkylthio,
      - Hydroxamino,
      - Hydroximino,
      - Sulfamoyl,
      - Sulfo,
      - Carboxamido,
      - Formyl,
      - Hydrazono,
      - Imino,
      - einen Rest CONR⁹R¹⁰,
      - durch bis zu sechs Hydroxy oder
      - durch bis zu fünf (C₁-C₈)-Alkanoyloxy substituiert ist;
   - mono-, bi- oder tricyclisches (C₃-C₁₈)-Cycloalkyl,
   - (C₃-C₁₈)-Cycloalkyl-(C₁-C₆)-alkyl,
      wobei der Cycloalkylteil jeweils gegebenenfalls durch einen oder zwei gleiche oder verschiedene Reste aus der Reihe
      - F, Cl, Br, I,
      - Carboxy,
      - Carbamoyl,
      - Carboxymethoxy,
      - Hydroxy,
      - (C₁-C₇)-Alkoxy,
      - (C₁-C₇)-Alkyl
      - (C₁-C₇)-Alkyloxycarbonyl,
      - Amino,
      - (C₁-C₆)-Alkylamino-(C₁-C₆)-alkyl,
      - Di-(C₁-C₆)-Alkylamino-(C₁-C₆)-alkyl,
      - Amidino,
      - Hydroxamino,
      - Hydroximino,
      - Hydrazono,
      - Imino,
      - Guanidino,
      - (C₁-C₆)-Alkoxysulfonyl,
      - (C₁-C₆)-Alkoxysulfinyl,
      - (C₁-C₆)-Alkoxycarbonylamino,
      - (C₆-C₁₂)-Aryl-(C₁-C₄)-alkoxycarbonylamino,
      - (C₁-C₇)-Alkylamino,
      - Di-(C₁-C₇)-alkylamino und
      - Trifluormethyl substituiert ist;
   - (C₆-C₁₄)-Aryl,
   - (C₆-C₁₄)-Aryl-(C₁-C₆)-alkyl,
   - (C₆-C₁₄)-Aryloxy-(C₁-C₆)-alkyl oder
   - (C₆-C₁₄)-Aryl-(C₃-C₈)-cycloalkyl,
      worin der Arylteil jeweils gegebenenfalls durch einen, zwei oder drei gleiche oder verschiedene Reste aus der Reihe
      - F, Cl, Br, I,
      - Hydroxy,
      - Mono-, Di- oder Trihydroxy-(C₁-C₄)-alkyl,
      - Trifluormethyl,
      - Formyl,
      - Carboxamido,
      - Mono- oder Di- (C₁-C₄)-alkylaminocarbonyl,
      - Nitro,
      - (C₁-C₇)-Alkoxy,
      - (C₁-C₇)-Alkyl,
      - (C₁-C₇)-Alkoxycarbonyl,
      - Amino,
      - (C₁-C₇)-Alkylamino,
      - Di-(C₁-C₇)-alkylamino,
      - Carboxy,
      - Carboxymethoxy,
      - Amino-(C₁-C₇)-alkyl,
      - (C₁-C₇)-Alkylamino-(C₁-C₇)-alkyl,
      - Di-(C₁-C₇)-alkylamino-(C₁-C₇)-alkyl,
      - (C₁-C₇)-Alkoxycarbonylmethoxy,
      - Carbamoyl,
      - Sulfamoyl,
      - (C₁-C₇)-Alkoxysulfonyl,
      - (C₁-C₈)-Alkylsulfonyl,
      - Sulfo-(C₁-C₈)-alkyl,
      - Guanidino-(C₁-C₈)-alkyl und
      - (C₁-C₆)-Alkoxycarbonylamino substituiert ist;
   - Het,
   - Het-(C₁-C₆)-alkyl,
   - Het-(C₃-C₈)-cycloalkyl,
   - Het-(C₃-C₈)-cycloalkyl-(C₁-C₄)-alkyl,
   - Het-(C₃-C₈)-cycloalkoxy-(C₁-C₄)-alkyl,
   - Het-thio-(C₁-C₆)-alkyl,
   - Het-thio-(C₃-C₈)-cycloalkyl,
   - Het-thio-(C₃-C₈)-cycloalkyl-(C₁-C₄)-alkyl,
      wobei Het jeweils für den Rest eines 5- bis 7-gliedrigen monocyclischen oder 8- bis 10-gliedrigen bicyclischen Ringsystems steht, das benzanelliert, aromatisch, teil- oder vollständig hydriert sein kann, das als Heteroelemente einen, zwei, drei oder vier verschiedene Reste aus der Gruppe N, O, S, NO, SO, SO₂ enthalten kann, das mit 1 bis 6 Hydroxy substituiert sein kann und das gegebenenfalls wie bei (C₆-C₁₄)-Aryl unter b₁) definiert und/oder mit Oxo mono-, di- oder trisubstituiert ist, oder einen Rest NR⁹R¹⁰ bedeutet oder,
b₂)
   - einen Rest der Formel VI

      R^{4a} - W (VI)

      worin R^{4a} wie R⁴ unter b₁) definiert ist und W für -CO-, -CS-, O-CO-, -SO₂-, -SO-, -S-, -NHSO₂-, -NHCO-, -CH(OH)-, -N(OH)- oder -CO-V-, wobei V ein Peptid mit insgesamt 1 bis 10 Amino-, Imino- und/oder Azaaminosäuren bedeutet, steht; oder worin R⁴ zusammen mit R⁸ und den diese tragenden Atomen mono-oder bicyclische, gesättigte oder teilweise ungesättigte Ringsysteme mit 5-12 Ringgliedern bilden, die außer Kohlenstoff noch 1 Schwefelatom enthalten können, welches gegebenenfalls zum Sulfoxid oder Sulfon oxidiert sein kann;
b₃)
   - einen Glycosylrest, bevorzugt einen Glucofuranosyl- oder Glucopyranosyl-Rest, der sich von natürlich vorkommenden Aldotetrosen, Aldopentosen, Aldohexosen, Ketopentosen, Ketohexosen, Desoxyaldosen, Aminoaldosen und Oligosacchariden sowie deren Stereoisomeren ableitet; oder
b₄)
   - eine Aminoschutzgruppe;

   - R⁵: - Wasserstoff oder
   - (C₁-C₈)-Alkyl bedeutet, oder
   - zusammen mit R⁶ und den diesen Rest tragenden Atomen mono- oder bicyclische, gesättigte oder teilweise ungesättigte Ringsysteme mit 5-12 Ringgliedern bildet;
   - R⁶: - wie R⁴ unter b₁) definiert ist;
   - für Hydroxy oder (C₁-C₄)-Alkanoyloxy steht; oder
   - zusammen mit R⁷ und den diesen Rest tragenden Atomen cyclische, gesättigte oder teilweise ungesättigte Ringsysteme mit 3 bis 12 Ringgliedern bildet; oder
   - zusammen mit R⁸ und den diese tragenden Atomen ein mono- oder bicyclisches, gesättigtes oder teilweise ungesättigtes Ringsystem mit 5-12 Ringgliedern bildet, das außer Kohlenstoff noch 1 Schwefelatom enthalten kann, welches gegebenenfalls zum Sulfoxid oder Sulfon oxidiert sein kann; oder 1 Stickstoffatom enthalten kann, wobei das Ringsystem gegebenenfalls durch Amino substituiert sein kann;
   - R⁷: - Wasserstoff oder
   - (C₁-C₆)-Alkyl bedeutet;
   - R⁸: - Wasserstoff,
   - Hydroxy,
   - (C₁-C₄)-Alkanoyloxy oder
   - (C₁-C₈)-Alkyl bedeutet;
   - R⁹ und R¹⁰: - Wasserstoff,
   - (C₁-C₈)-Alkyl, das durch
      - Amino,
      - (C₁-C₄)-Alkylamino,
      - Di-(C₁-C₄)-alkylamino,
      - Mercapto,
      - Carboxy,
      - Hydroxy oder
      - (C₁-C₄)-Alkoxy substituiert sein kann,
   - (C₃-C₇)-Cycloalkyl,
   - (C₁-C₄)-Alkoxycarbonyl,
   - (C₆-C₁₄)-Aryl, (C₆-C₁₄)-Aryl-(C₁-C₄)-alkoxycarbonyl, die im Arylteil wie bei R⁴ beschrieben, substituiert sein können,
   - Het oder
   - Het-(C₁-C₄)-alkyl, wobei Het wie bei R⁴ beschrieben definiert ist, bedeuten oder wobei

R⁹ und R¹⁰ zusammen mit dem sie tragenden Stickstoffatom monocyclische oder bicyclische, gesättigte, teilweise ungesättigte oder aromatische Ringsysteme bilden, die als Ringglieder neben Kohlenstoff noch 1 oder weitere 2 Stickstoffatome, 1 Schwefelatom oder 1 Sauerstoffatom enthalten und durch (C₁-C₄)-Alkyl substituiert sein können, wobei in den vorstehenden Verbindungen der Formel I eine oder mehrere Amidgruppen (-CONH-) der Hauptkette ersetzt sein können durch -CH₂NR¹¹-, -CH₂S-, -CH₂O-, -OCH₂-, -CH₂CH₂-, -CH=CH-(cis und trans), -COCH₂-, -CH(OH)CH₂-, -CH₂SO-, -CH₂SO₂-, -COO-, -P(O)(OR¹²)CH₂- und -P(O)(OR¹²)NH-, oder auch durch eine Amidgruppe mit umgekehrter Polarität (-NHCO-);
worin R¹¹ und R¹² unabhängig voneinander stehen für
- Wasserstoff oder
- (C₁-C₄)-Alkyl;
sowie deren Enantiomere und physiologisch verträgliche Salze, dadurch gekennzeichnet, daß homochirale α-Aminoaldehyde der Formel VII 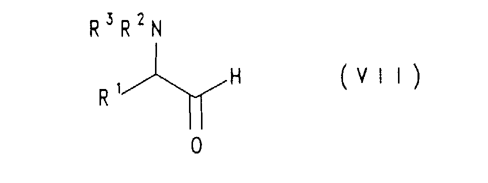 worin R¹, R² und R³ wie oben definiert sind, mit einem in situ aus VCl₃, THF und Zinkstaub erhaltenen Vanadium-Komplex in THF und unter Züsatz eines Komplexbildners behandelt werden, wobei eine gleichzeitige Kontrolle über alle vier Chiralitätszentren vorliegt.

Bevorzugt werden Verbindungen der Formel I hergestellt, worin
- R¹: einen Seitenkettenrest der α-Aminosäuren Gly, Ala, Val, Leu, Ile, Ser, Thr, Cys, Met, Pro, Lys, Arg, His, Asp, Asn, Glu, Gln, Phe, Tyr, Trp oder Cha bedeutet;
- R² und R³: gleich oder verschieden sind und
a)
   - Wasserstoff
b)
   - einen Rest der Formel II
      worin o und p = 0,
      n = 0 oder 1 ist und
      E für eine der oben genannten α-Aminosäuren steht,
      D für R⁴ oder einen Rest der Formeln III oder IV steht, worin R⁴

   b₁₎
      - Wasserstoff
      - (C₁-C₉)-Alkyl, das gegebenenfalls einfach oder zweifach ungesättigt ist und das gegebenenfalls durch bis zu 3 gleiche oder verschiedene Reste aus der Reihe
         - Hydroxy,
         - (C₁-C₇)-Alkoxy,
         - Carbamoyl,
         - (C₁-C₈)-Alkanoyloxy,
         - (C₁-C₇)-Alkoxycarbonyl,
         - F, Cl,
         - Amino,
         - (C₁-C₇)-Alkylamino,
         - Di-(C₁-C₇)-Alkylamino,
         - (C₁-C₆)-Alkoxycarbonylamino,
         - (C₇-C₁₅)-Aralkoxycarbonyl,
         - (C₇-C₁₅)-Aralkoxycarbonylamino,
         - Phenyl-(C₁-C₄)-alkoxy,
         - 9-Fluorenylmethoxycarbonylamino,
         - (C₁-C₆)-Alkylsulfonyl,
         - (C₁-C₆)-Alkylsulfinyl,
         - (C₁-C₆)-Alkylthio subsitutiert ist,
      - (C₆-C₁₄)-Aryl,
      - (C₆-C₁₄)-Aryl-(C₁-C₆)-alkyl oder
      - (C₆-C₁₄)-Aryloxy-(C₁-C₆)-alkyl, worin der Arylteil jeweils gegebenenfalls durch einen, zwei oder drei gleiche oder verschiedene Reste aus der Reihe der oben genannten bevorzugten Substituenten von (C₁-C₉)-Alkyl substituiert sein kann,
   b₂₎ - einen Rest der Formel VI, worin
      R^{4a} wie R⁴ unter b₁₎ definiert ist und W für -CO-, O-CO-, -SO₂-, -SO-, -S-, -NHCO-, -CH(OH)- steht;
b₄) - eine Aminoschutzgruppe Fmoc, Z oder Boc bedeutet,

- R⁵ und R⁷: Wasserstoff bedeuten,
- R⁶: - wie R⁴ definiert ist, und
- R⁸: - Wasserstoff,
- Hydroxy,
- (C₁-C₄)-Alkanoyloxy oder
- (C₁-C₈)-Alkyl bedeutet.

Bevorzugt sind ferner Verbindungen der Formel I, worin einer der Reste R² oder R³ Wasserstoff bedeutet.

Weiterhin sind Verbindungen der Formel I mit SRRS-Konfiguration (wenn Aldehyde der Formel VII mit (S)-Konfiguration eingesetzt werden) bzw. Verbindungen der Formel I mit der RSSR-Konfiguration (wenn Aldehyde der Formel VII mit (R)-Konfiguration eingesetzt werden) bevorzugt.

Ganz besonders bevorzugt sind Verbindungen der Formel I, worin
- R¹: - einen Seitenkettenrest der α-Aminosäuren Ala, Val, Leu, lle, Pro, Phe, Cha oder Tyr bedeutet,
- R² und R³: gleich oder verschieden sind und

a)
   - Wasserstoff
b)
   - einen Rest der Formel II, worin
      o und p = 0 sind,
      n 0 oder 1 bedeutet und
      E für Ala, Val, Leu, Ile, Pro, Phe, Cha oder Tyr steht;
      D für R⁴ oder einen Rest der Formel IV steht mit R⁴
b₁₎
   - Wasserstoff,
   - (C₁-C₄)-Alkyl,
   - Phenyl oder Naphthyl,
   - Phenylmethyl oder Naphthylmethyl,
b₂)
   - Rest der Formel VI, worin R^{4a} wie R⁴ unter b₁₎ definiert ist und W für -CO-, -O-CO-, -SO₂-, -SO-, -S-, -NHCO-, -CH(OH)- steht, oder
b₄)
   - eine Aminoschutzgruppe Fmoc, Z oder Boc bedeuten,

- R⁵, R⁷, R⁸: Wasserstoff bedeutet, und
- R⁶: wie R⁴ unter b₁) definiert ist.

Ganz besonders bevorzugt sind weiterhin Verbindungen der Formel I mit der SRRS-Konfiguration, erhalten unter Einsatz von Aldehyden der Formel VII mit (S)-Absolutkonfiguration. Diese Aussage gilt nur unter der Voraussetzung, daß die Gruppe R¹ niedrigere Cahn-Ingold-Prelog-Priorität als die Gruppe -CH(OH)-CH(OH)-hat.

α-Aminosäuren können, falls chiral, in der S- oder R-Form vorliegen. Sie entsprechen nachstehender Formel VIII 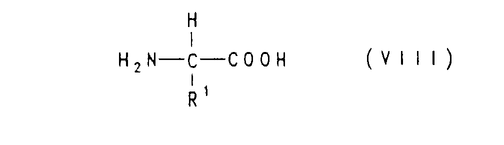 und unterscheiden sich lediglich im Rest R¹ der Seitenkette. Beispielhaft sind nachstehend einige natürliche und nicht-natürliche α-Aminosäuren im Dreibuchstabencode genannt:

Aad, Abu, ABz, 2ABz, Ach, Acp, Adpd, Ahb, Aib, Ala, Alg, All, Ama, Amt, Ape, Apm, Apr, Arg, Asn, Asp, Asu, Aze, Azi, Bai, Bph, Can, Cha, Cit, Cys, (Cys)₂, Cyta, Daad, Dab, Dadd, Dap, Dapm, Dasu, Djen, Dpa, Dtc, Fel, Gln, Glu, Gly, Guv, hAla, hArg, hCys, hGln, hGlu, His, hIle, hLeu, hLys, hMet, hPhe, hPro, hSer, hThr, hTrp, Hyl, Hyp, 3Hyp, Ile, Ise, Iva, Kyn, Lant, Lcn, Leu, Lsg, Lys, Met, Mim, Min, nArg, Nle, Nva, Oly, Orn, Pan, Pec, Pen, Phe, Phg, Pic, Pro, Pse, Pya, Pyr, Pza, Qin, Ros, Sar, Sec, Sem, Ser, Thi, Thr, Thy, Thx, Tia, Tle, Tly, Trp, Trta, Tyr, Val, Nal, Tbg, Npg, Chg, Thia, Cha (siehe z.B. Houben-Weyl, "Methoden der organischen Chemie" Band XV/1 und 2, Stuttgart 1974). Falls in Einzelverbindungen nicht anders angegeben, steht die Abkürzung eines Aminosäurerestes ohne einen Stereodeskriptor für den Rest in der L-Form, welche üblicherweise der S-Konfiguration entspricht.

Unter einer Iminosäure werden allgemein natürliche oder unnatürliche Aminosäuren verstanden, deren Aminogruppe monosubstituiert ist. Besonders seien in diesem Zusammenhang Verbindungen genannt, die durch (C₁-C₈)-Alkyl substituiert sind. Ferner kommen Heterocyclen aus der folgenden Gruppe in Betracht:
Pyrrolidin-2-carbonsäure; Piperidin-2-carbonsäure; 1,2,3,4-Tetrahydroisochinolin-3-carbonsäure; Decahydroisochinolin-3-carbonsäure; Octahydroindol-2-carbonsäure; Decahydrochinolin-2-carbonsäure; Octahydrocyclopenta[b]pyrrol-2-carbonsäure; 2-Azabicyclo[2.2.2]octan-3-carbonsäure; 2-Azabicyclo[2.2.1]heptan-3-carbonsäure; 2-Azabicyclo[3.1.0]hexan-3-carbonsäure; 2-Azaspiro[4.4]nonan-3-carbonsäure; 2-Azaspiro[4.5]decan-3-carbonsäure; Spiro[(bicyclo[2.2.1]heptan)-2,3-pyrrolidin-5-carbonsäure]; Spiro[(bicyclo[2.2.2]octan)-2,3-pyrrolidin-5-carbonsäure]; 2-Azatricyclo[4.3.0.1^{6.9}] decan-3-carbonsäure; Decahydrocyclohepta[b]pyrrol-2-carbonsäure; Decahydrocycloocta[b]pyrrol-2-carbonsäure; Octahydrocyclopenta[c]pyrrol-2-carbonsäure; Octahydroisoindol-1-carbonsäure; 2,3,3a,4,6a-Hexahydrocyclopenta[b] pyrrol-2-carbonsäure; 2,3,3a,4,5,7a-Hexahydroindol-2-carbonsäure; Tetrahydrothiazol-4-carbonsäure; Isoxazolidin-3-carbonsäure; Pyrazolidin-3-carbonsäure; Hydroxyprolin-2-carbonsäure, die alle gegebenenfalls substituiert sein können:

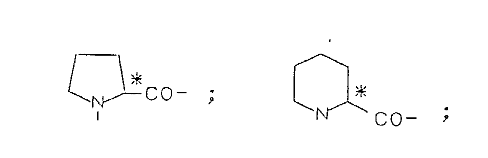

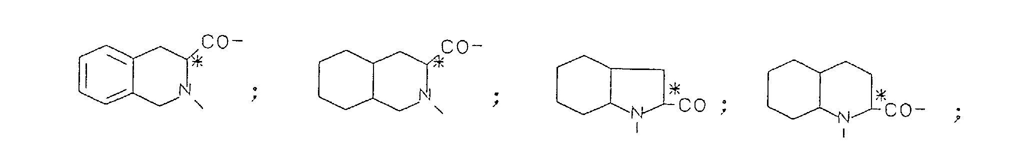

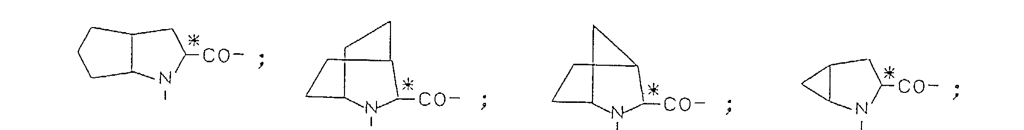

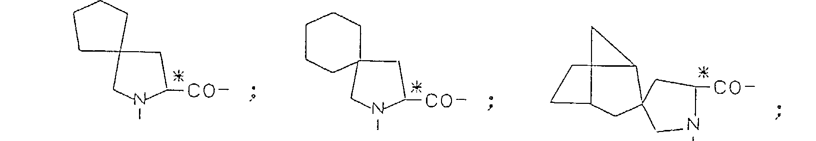

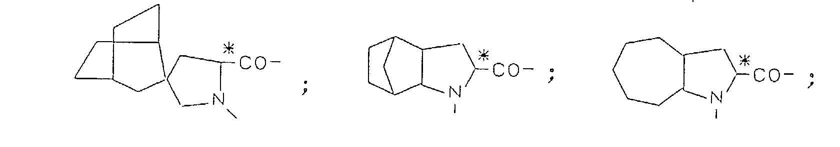

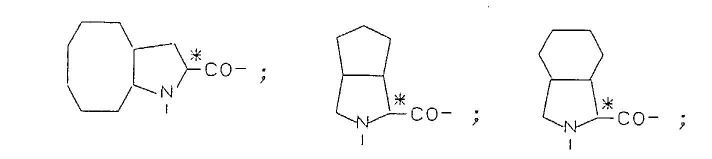

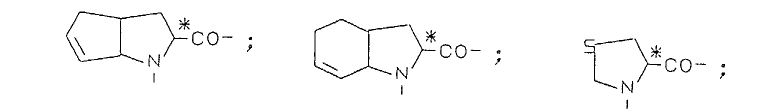

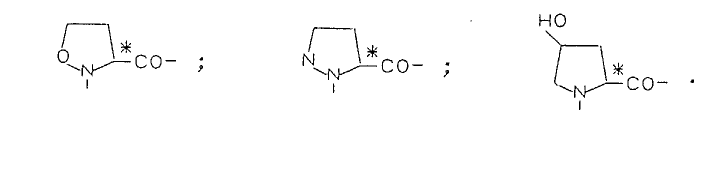

Azaaminosäuren sind von natürlichen oder nicht-natürlichen Aminosäuren abgeleitet, wobei der Zentralbaustein -CHR- bzw. -CH₂- durch -NR- bzw. -NH-ersetzt ist.

Einen Überblick über die Synthesen, insbesondere der nicht-natürlichen optisch aktiven α-Amino- und Iminosäuren gibt R.M. Williams in "Synthesis of Optically Active α-Aminoacids", Pergamon Press, Oxford 1989.

Die in dieser Beschreibung benutzte Nomenklatur folgt der allgemeinen Praxis bei Aminosäuren, das heißt, die Aminogruppe steht links, die Carboxygruppe rechts von jeder Aminosäure. Entsprechendes gilt für Iminosäuren und Azaaminosäuren.

Aminoschutzgruppen sind in R.Geiger und W.König "The Peptides" Volume 3 "Protection of Functional Groups in Peptide Synthesis" , E.G.Gross, J.Meienhofer Edit., Academic Press, New York (1981), insbesondere Seiten 7 - 46, beschrieben. Einige sind nachfolgend beispielhaft angegeben:

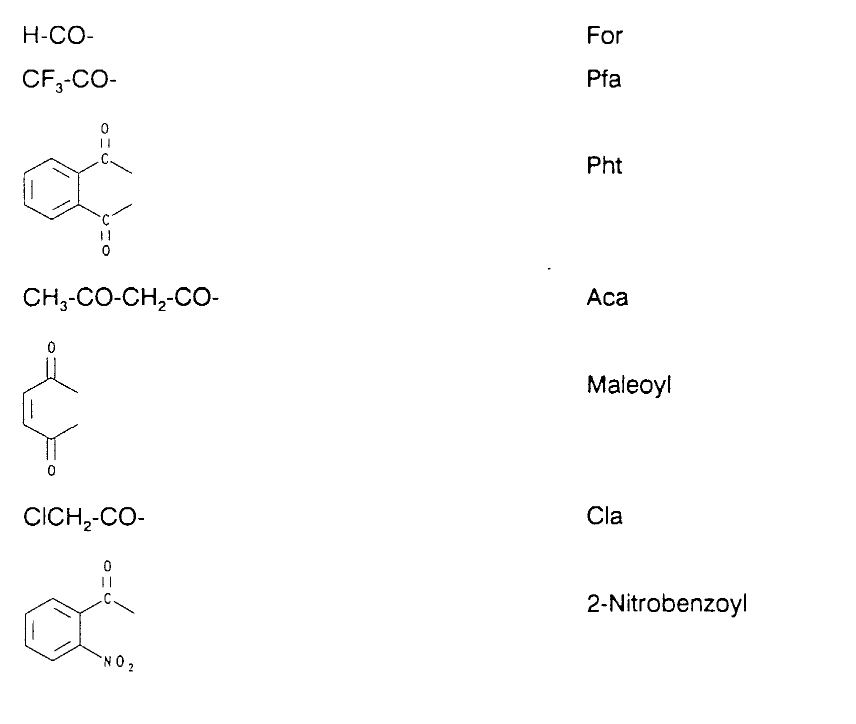

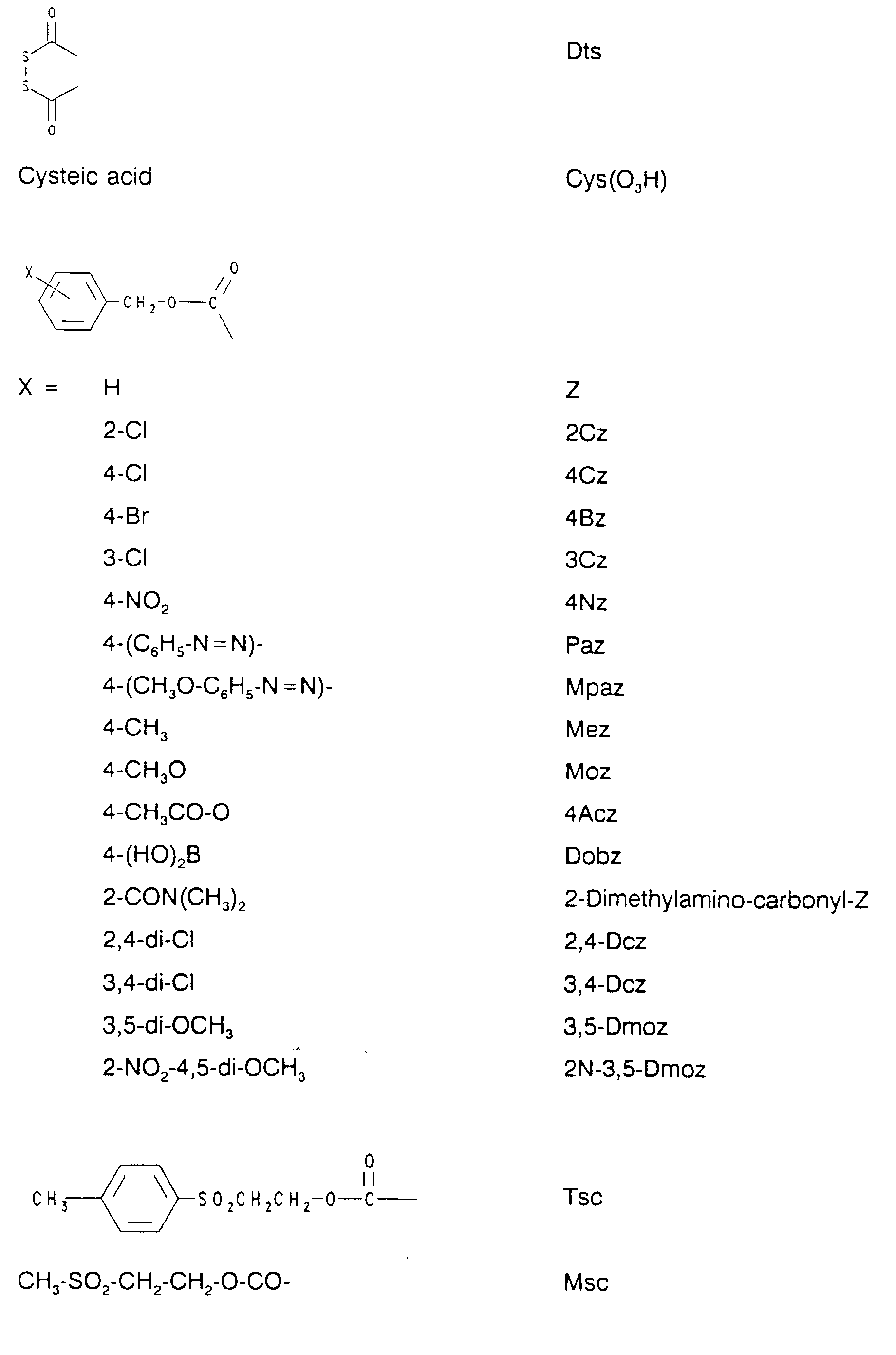

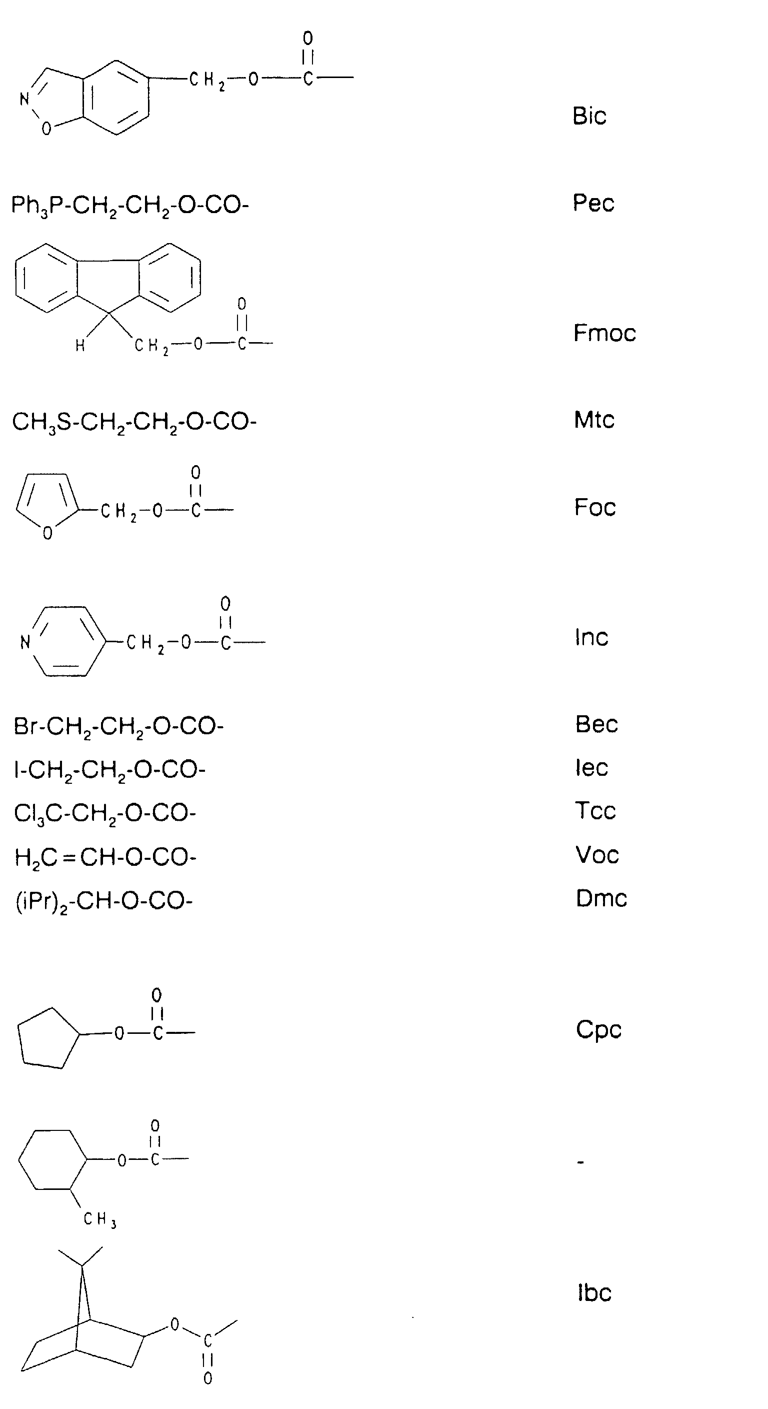

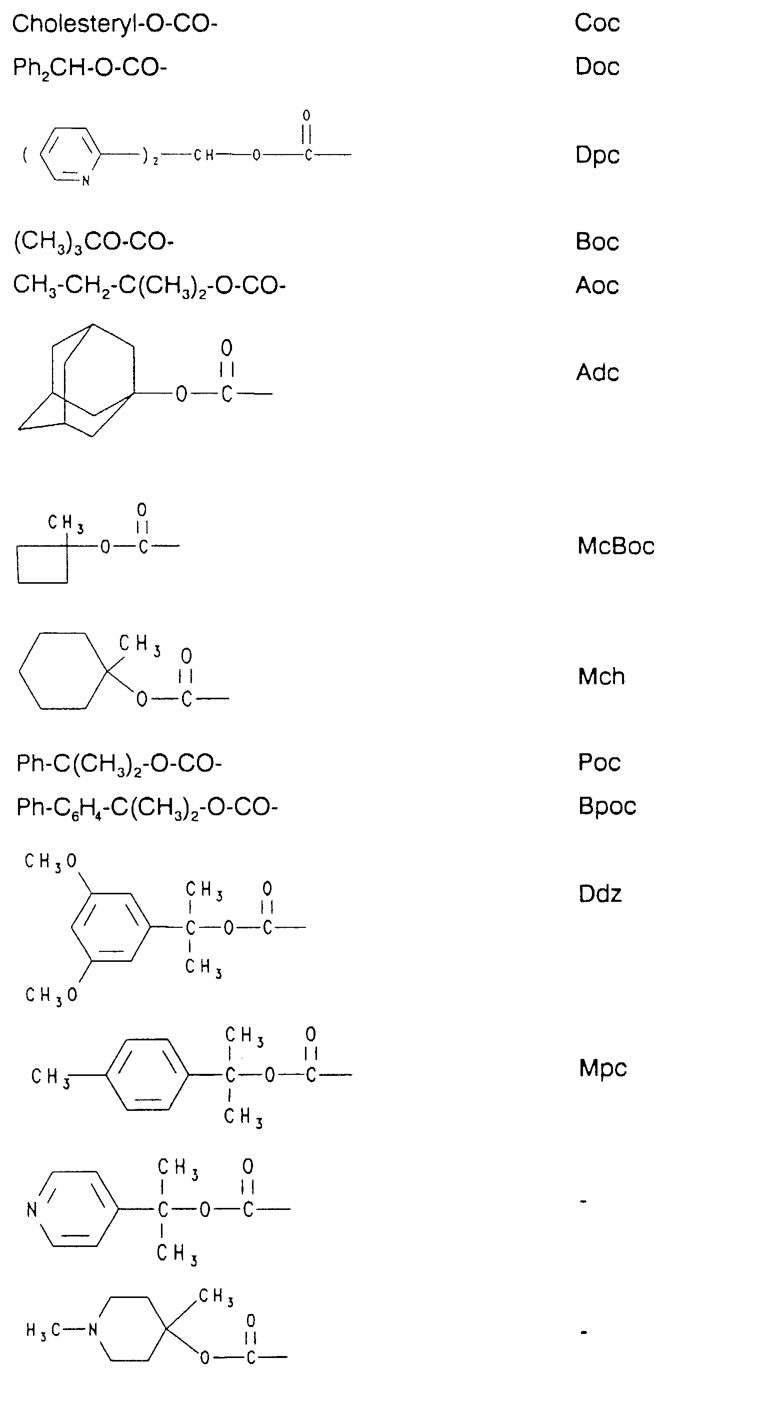

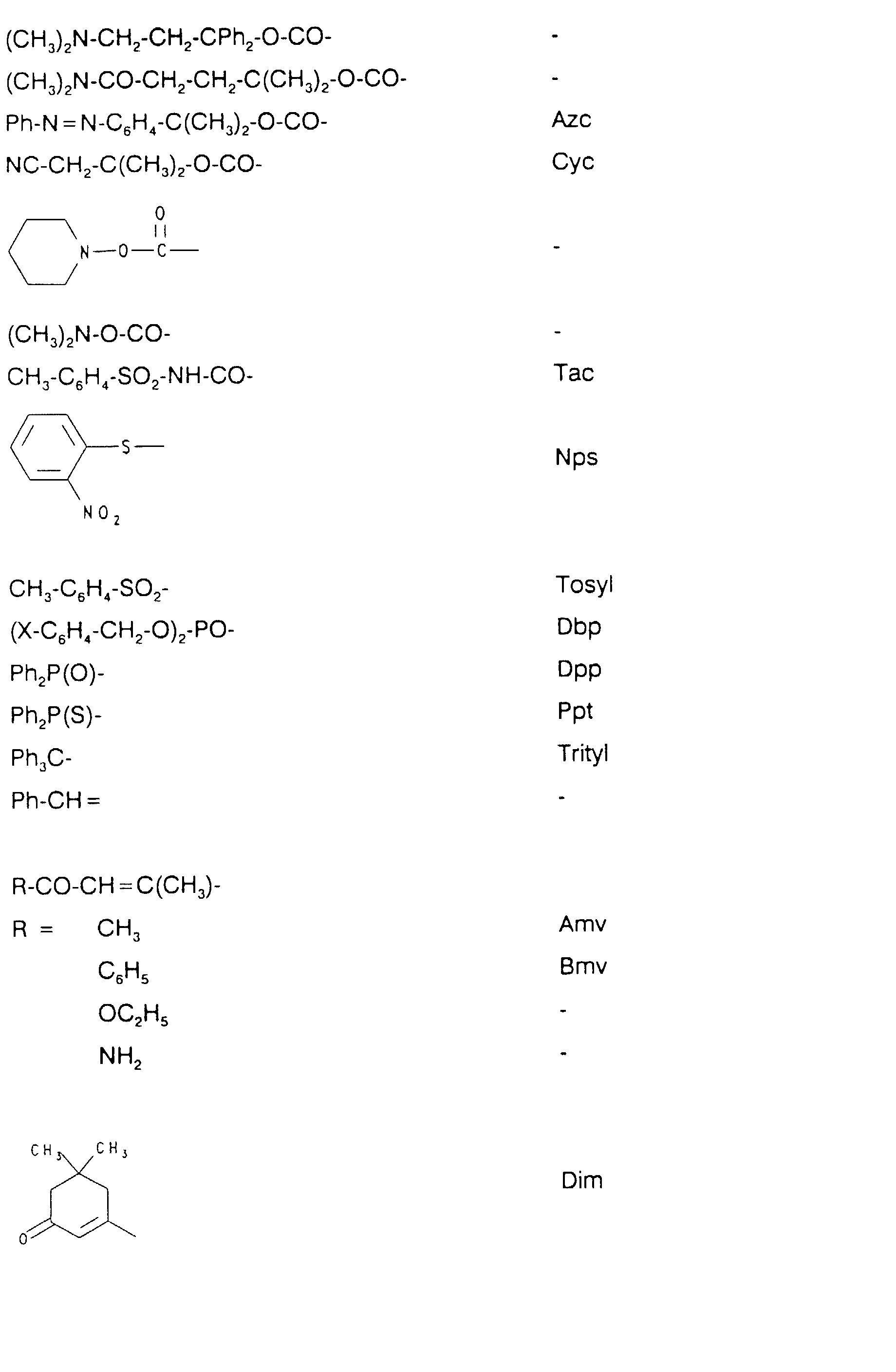

Funktionelle Gruppen in den Seitenketten der Amino-, Imino- oder Azaaminosäuren können beispielsweise wie folgt geschützt werden:
a) die Guanidinogruppe (z.B. von Arginin) kann gemäß Geiger/König in E. Gross, J. Meinhofer ("The Peptides- Protection of Functional Groups in Peptide Synthesis", Academic Press, New York, 1981), S. 60 - 70 geschützt werden;
b) der Aminostickstoff (z.B. von Lysin) kann gemäß S. 7 - 49 geschützt werden;
c) der Imidazolstickstoff (z.B. von Histidin) kann gemäß S. 70 - 80 geschützt werden;
d) der Pyrazolylstickstoff (z.B. von β-3-Pyrolylalanin) kann gemäß S. 81 - 82 geschützt werden;
e) der Indolstickstoff (z.B. von Tryptophan) kann gemäß S. 82 - 84 geschützt werden;
f) die Carboxylgruppe (z.B. von Asparaginsäure) kann gemäß S. 102 - 132 geschützt werden;
g) die Sulfhydrylgruppe (z.B. von Cystein) kann gemäß S. 137 - 169 geschützt werden;
h) die Hydroxylgruppe (z.B. von Serin, Threonin, Tyrosin) kann gemäß S. 170 - 201 geschützt werden;
i) im Falle, daß R² einer Peptidgruppe entspricht, können, wo erforderlich, peptidische Amidstickstoffe gemäß S. 52-59 geschützt werden.

Glycosylreste wie vorstehend beschrieben leiten sich insbesondere von natürlichen, in Mikroorganismen, Pflanzen, Tieren oder Menschen vorkommenden D- oder L-Monosacchariden wie Ribose (Rib), Arabinose (Ara), Xylose (Xyl), Lyxose (Lyx), Allose (All), Altrose (Alt), Glucose (Glc), Mannose (Man), Gulose (Gul), Idose (Ido), Galactose (Gal), Talose (Tal), Erythrose (Ery), Threose (Thr), Psicose (Psi), Fructose (Fru), Sorbose (Sor), Tagatose (Tag), Xylulose (Xyu), Fucose (Fuc), Rhamnose (Rha), Olivose (Oli), Oliose (Olo), Mycarose (Myc), Rhodosamin (RN), N-Acetyl-glucosamin (GlcNAc), N-Acetyl-galactosamin (GalNAc), N-Acetyl-mannosamin (ManNAc) oder Disacchariden, wie Maltose (Mal), Lactose (Lac); Cellobiose (Cel), Gentibiose (Gen), N-Acetyl-lactosamin (LacNAc), Chitobiose (Chit), β-Galactopyranosyl-(1-3)-N-acetylgalactosamin und β-Galactopyranosyl-(1-3)- oder (1-4)-N-acetyl-glucosamin, sowie deren synthetischen Derivaten, wie 2-Desoxy-, 2-Amino-, 2-Acetamido- oder 2-Halogeno-, bevorzugt Bromo- und Jodo-Zucker ab.

Alkyl kann geradkettig oder verzweigt sein. Entsprechendes gilt für davon abgeleitete Reste, wie z.B. Alkoxy, Alkylthio, Alkylamino, Dialkylamino, Alkanoyl und Aralkyl.

Unter Cycloalkyl werden auch alkylsubstituierte Reste, wie z.B. 4-Methylcyclohexyl oder 2,3-Dimethylcyclopentyl verstanden.

(C₆-C₁₄)-Aryl ist beispielsweise Phenyl, Naphthyl, Biphenylyl oder Fluorenyl; bevorzugt sind Phenyl und Naphthyl. Entsprechendes gilt für davon abgeleitete Reste, wie z.B. Aryloxy, Aroyl, Aralkyl und Aralkoxy. Unter Aralkyl versteht man einen mit (C₁-C₆)-Alkyl verknüpften unsubstituierten oder substituierten (C₆-C₁₄)-Aryl-Rest, wie z.B. Benzyl, 1- und 2-Naphthylmethyl, wobei Aralkyl jedoch nicht auf die genannten Reste beschränkt wäre.

Reste Het im Sinne vorstehender Definition sind Pyrrolyl, Furyl, Thienyl, Imidazolyl, Pyrazolyl, Oxazolyl, Isoxazolyl, Thiazolyl, Isothiazolyl, Tetrazolyl, Pyridyl, Pyrazinyl, Pyrimidinyl, Indolyl, Isoindolyl, Indazolyl, Phthalazinyl, Chinolyl, Isochinolyl, Chinoxalinyl, Chinazolinyl, Cinnolinyl, β-Carbolinyl, oder ein benzanelliertes, cyclopenta-, cyclohexa- oder cyclohepta-anelliertes Derivat dieser Reste.

Diese Heterocyclen können an einem Stickstoffatom durch Oxido; (C₁-C₇)-Alkyl, z.B. Methyl oder Ethyl; Phenyl; Phenyl-(C₁-C₄)-alkyl, z.B. Benzyl; und/oder an einem oder mehreren Kohlenstoffatomen durch (C₁-C₄)-Alkyl, z.B. Methyl; Phenyl; Phenyl-(C₁-C₄)-alkyl, z.B. Benzyl; Halogen; Hydroxy; (C₁-C₄)-Alkoxy, z.B. Methoxy; Phenyl-(C₁-C₄)-alkoxy, z.B. Benzyloxy; oder Oxo substituiert und teilweise oder vollständig gesättigt sein.

Derartige Reste sind beispielsweise 2- oder 3-Pyrrolyl; Phenyl-pyrrolyl, z.B. 4- oder 5-Phenyl-2-pyrrolyl; 2-Furyl; 2-Thienyl; 4-Imidazolyl; Methyl-imidazolyl, z.B. 1-Methyl-2-, -4- oder -5-imidazolyl; 1,3-Thiazol-2-yl; 2-, 3- oder 4-Pyridyl; 2-, 3- oder 4-Pyridyl-N-oxid; 2-Pyrazinyl; 2-, 4- oder 5-Pyrimidinyl; 2-, 3- oder 5-Indolyl; substituiertes 2-Indolyl, z.B. 1-Methyl-, 5-Methyl-, 5-Methoxy-, 5-Benzyloxy-, 5-Chlor- oder 4,5-Dimethyl-2-indolyl; 1- Benzyl- 2- oder -3-indolyl; 4,5,6,7-Tetrahydro-2-indolyl; Cyclohepta[b]-5-pyrrolyl; 2-, 3- oder 4-Chinolyl; 1-, 3- oder 4-Isochinolyl; 1-Oxo-1,2-dihydro-3-isochinolyl, 2-Chinoxalinyl; 2-Benzofuranyl; 2-Benzoxazolyl; Benzothiazolyl; Benz[e]indol-2-yl oder β-Carbolin-3-yl.

Teilhydrierte oder vollständig hydrierte heterocyclische Ringe sind beispielsweise Dihydropyridinyl; Pyrrolidinyl, z.B. 2-, 3- oder 4-N-Methylpyrrolidinyl; Piperazinyl; Morpholino; Thiomorpholino; Tetrahydrothiophenyl; Benzodioxolanyl.

Halogen steht für Fluor, Chlor, Brom oder Jod, insbesondere für Fluor oder Chlor.

Unter Salzen von Verbindungen der Formel (I) sind insbesondere pharmazeutisch verwendbare oder nicht-toxische Salze zu verstehen.

Solche Salze werden beispielsweise von Verbindungen der Formel (I), welche saure Gruppen, z.B. Carboxy, enthalten, mit Alkali- oder Erdalkalimetallen gebildet, wie z.B. Na, K, Mg und Ca, sowie mit physiologisch verträglichen organischen Aminen, wie z.B. Triethylamin und Tris-(2-hydroxy-ethyl)-amin.

Verbindungen der Formel (I), welche basische Gruppen, z.B. eine Aminogruppe oder eine Guanidinogruppe enthalten, bilden mit anorganischen Säuren, wie z.B. Salzsäure, Schwefelsäure oder Phosphorsäure und mit organischen Carbon- oder Sulfonsäuren, wie z.B. Essigsäure, Citronensäure, Benzoesäure, Maleinsäure, Fumarsäure, Weinsäure und p-Toluolsulfonsäure, Salze.

Eine Ausführungsform des erfindungsgemäßen Verfahrens ist dadurch gekennzeichnet, daß man Aldehyde der Formel VII mit Hilfe des eines in situ aus VCl₃, THF und Zinkstaüb Vanadium(II)komplexes in THF und unter Züsatz eines Komplexbildners im Temperaturbereich von -78 °C bis zur Siedetemperatur stereoselektiv reduktiv zu den Verbindungen der Formel I dimerisiert.

Eine bevorzugte Ausführungsform zur Herstellung der Verbindungen der Formel I mit der obengenannten bevorzugten Konfiguration ist dadurch gekennzeichnet, daß man VCl₃ in einer schutzgasgespülten Apparatur (z.B. N₂ oder Argon) in THF bei Temperaturen von -78 °C bis Siedetemperatur, bevorzugt von 0 °C bis zur Siedetemperatur vorlegt und sukzessive mit 0,5 bis 1,0, bevorzugt 0,5 bis 0,7 Equivalenten Zinkstaub und 0 bis 9 Equivalenten eines Komplexbildners, wie DMF, HMPA, DMSO, 1,3-Dimethylimidazolidin-2-on, DABCO, TMEDA, EDTA, Nitrilotriessigsäure, Triethanolamin, Glyme, Diglyme, Triglyme, Ethylenglycol, Diethylenglycol, Kronenether, Kryptanden, bevorzugt mit 2 bis 7 Equivalenten HMPA, und mit 0,2 bis 1,0, vorzugsweise 0,4 bis 0,6 Equivalenten Aldehyd der allgemeinen Formel VII versetzt und bis zur Reaktionsvervollständigung nach DC-Kontrolle unter Schutzgasatmosphäre (z.B. N₂ oder Argon) bei der jeweiligen Anfangstemperatur rührt. Zur Aufarbeitung wird die Reaktionstemperatur auf Zimmertemperatur gestellt und die Mischung mit der wäßrigen Lösung eines Komplexbildners, vorzugsweise 10 - 30%iger wäßriger Zitronensäure- oder Tartratlösung versetzt. Nach der Phasentrennung wird die wäßrige Phase mit im Reaktionsansatz verwendetem Lösungsmittel oder ersatzweise mit einem mit Wasser nicht mischbaren, organischen Lösungsmittel extrahiert, die vereinigten organischen Phasen getrocknet, filtriert und zur Trockne eingedampft, wonach das Rohprodukt in Ausbeuten von 20% bis 100% d.Th. anfällt. Die Reinigung erfolgt bevorzugt durch Kristallisation oder Chromatographie an einer Kieselgelsäule oder entfällt aufgrund der ausreichenden Reinheit des anfallenden Rohproduktes.

Die besonders bevorzugten Verbindungen der Formel I in der SRRS-Konfiguration erhält man bevorzugt aus den α-Aminoaldehyden der Formel VII mit S-Konfiguration bei Raumtemperatur oder erhöhter Temperatur.

Optisch reine α-Aminoaldehyde der Formel VII werden in einfacher, literaturbekannter Weise aus Aminosäuren erhalten, z.B. wie nachfolgend näher erläutert.

Kommerziell erhältliche oder selbst synthetisierte Verbindungen der Formel IX 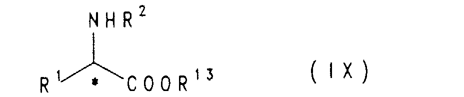 worin
- R¹³: H, (C₁-C₄)-Alkyl, (C₆-C₁₄)-Aryl oder (C₆-C₁₄)-Aryl-(C₁-C₆)-alkyl, insbesondere Methyl, Ethyl oder Benzyl bedeutet und
- R¹ und R²: wie oben definiert sind
(Houben Weyl 15/1 und 2, Stuttgart, 1974; V. Teetz, R. Geiger, H. Gaul, Tetrahedron Letters 25(40), 4479 (1984), A. Pictet, T. Spengler, Chem. Ber. 44,2030 (1911); R.M. Williams, "Synthesis of Optically Active α-Aminoacids", Pergamon Press, Oxford, 1989) werden analog zu literaturbekannten Methoden (M.W. Drewes, "The Syntheses and Stereoselektive Reactions of α-Aminoaldehydes", Inauguraldissertation, Fachbereich Chemie der Philipps-Universität, Marburg/Lahn 1988; und darin zitierte Lit.; N.G. Gaylord, "Reduction with Complex Metal Hydrides", Interscience Publishers, NY London, 1956; H. Schenker, Angew. Chemie 73, 81 (1961); C.F. Stanfield, J.E. Parker, P. Kanellis, J. Org. Chem.46, 4797 und 4799 (1981); K.E. Rittle, C.F. Homnick, B.E. Evans, J. Org. Chem.47, 3016 (1982); K. Haaf, C. Rüchardt, Chem. Ber.123, 635 (1990)) beispielsweise mit NaBH₄ (N.G. Gaylord, s.o.), BH₃·THF (K.E. Rittle, s.o.), oder LiAlH₄ (K. Haaf, s.o.) in inerten Lösungsmitteln, oder niederen Alkoholen, oder alkoholisch-wäßrigen Mischungen zu den Aminoalkoholen der Formel X reduziert, 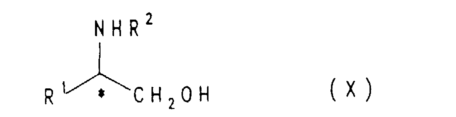 wobei
R¹ und R² wie oben definiert sind. Anschließend werden die so erhaltenen Verbindungen der Formel X nach bekannten Verfahren (Houben Weyl, s.o.; E. Gross, J. Meinhofer, Ed., "The Peptides - Protection of Functional Groups in Peptide Synthesis", Academic Press, New York, 1981; T.W. Greene, "Protective Groups in Organic Synthesis", John Wiley & Sons, NY Chichester Brisbane Toronto Singapore, 1980; Proceedings of European Peptide Symposium, Platja D'Aro, September 1990 (EP 0 460 446) zu den Verbindungen der Formel XI 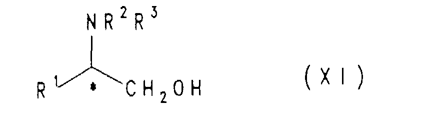 mit den genannten Bedeutungen für R¹ - R³ umsetzt und anschließend mit Pyridiniumdichromat (C.F. Stanfield, s.o.), CrO₃·Pyridin (K.E. Rittle, s.o.), insbesondere aber mit (COCl)₂ und DMSO und nach der Methode von Swern racemisierungsfrei zu den Aldehyden der Formel VII 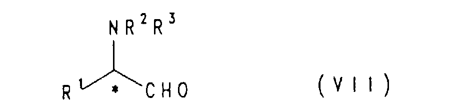 wobei R¹, R² und R³ wie oben definiert sind, oxidiert (K. Omura, A.K. Sharma, D. Swern, J. Org. Chem. 41, 957 (1976); D. Swern, S.L. Huang, A.J. Mancuso, J. Org. Chem. 43, 2480 (1978); A.J. Mancuso, D. Swern, Synthesis, 165 (1981)).

Eine zweite Variante besteht darin daß man Verbindungen der Formel IX analog zu den oben genannten literaturbekannten Synthesen zu Verbindungen der Formel XII 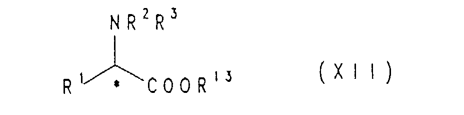 worin R¹, R², R³ und R¹³ wie oben definiert sind, umsetzt und diese - nach eventuell vorangehender racemisierungsfreier Verseifung eines Esters der Formel XII mit R¹³ ≠ H - beispielsweise nach der Methode von Weinreb (S. Nahm, S.M. Weinreb, Tetrahedron Letters 22, 3815 (1981)) durch Umsetzung mit N,O-Dimethylhydroxylamin zu den Verbindungen der Formel XIII umsetzt, 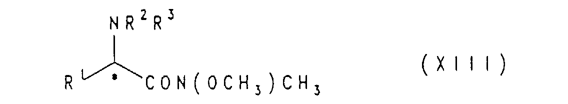 wobei R¹, R², R³ wie oben definiert sind. Die Amide der allgemeinen Formel XIII werden zum Beispiel nach der Methode von Castro (J.A. Fehrentz, B. Castro, Synthesis, 676 (1983); J.A. Fehrentz, B. Castro, Int. J. Peptide Protein Res. 26, 236 (1985)) durch die Reduktion mit LiAlH₄ direkt und racemisierungsfrei in die genannten Aldehyde der allgemeinen Formel VII überführt.

In einer dritten Variante werden Carbonsäuren der allgemeinen Formel XII (R¹³ = H) mit Thionylchlorid oder anderen geeigneten Halogenierungsmitteln in die entsprechenden Carbonsäurehalogenide der Formel XIV 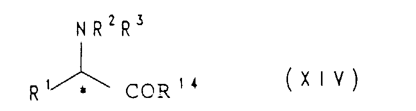 wobei
R¹ - R³ den obigen Definitionen entspricht und
R¹⁴ Cl, Br, I oder Reste gemischter Carbonsäureanhydride bedeutet, derivatisiert und nachfolgend mit H₂/Pd/BaSO₄ racemisierungsfrei zu den Aldehyden der allgemeinen Formel VII reduziert (analog: R.L. Johnson, J. Med. Chem. 25, 605 (1982)). Im Prinzip können Aldehyde aus Carbonsäuren und deren Derivaten auch mit anderen Methoden hergestellt werden, etwa durch Umsetzung mit einfachen und komplexen Metallhydriden, Metallcarbonylkomplexen, Silanen, Alkalimetallen, Formiaten oder photochemisch (Houben Weyl 7E3, 418ff, Stuttgart, 1983).

Im Gegensatz zu den bei Pedersen et al. beschriebenen Pinakolkopplungen (J.H. Freudenberger, s.o.; A.W. Konradi, S.F. Pedersen, J. Org. Chem. 55, 4506 (1990); P.M. Takahara, J.H. Freudenberger, A.W. Konradi, S.F. Pedersen, Tetrahedron Letters 30 (51), 7177 (1989); A.S. Raw, S.F. Pedersen, J. Org. Chem. 56, 830 (1991)) wird bei dem vorliegenden Verfahren eine gleichzeitige Kontrolle über vier Stereozentren ausgeübt. Beim Einsatz von optisch aktiven Ausgangsmaterialien werden dabei optisch aktive Kopplungsprodukte in hoher Ausbeute gewonnen. Ein weiterer Vorteil des hier beschriebenen Verfahrens ist die größere Selektivität des Reduktionsmittels für die aktivierte Aldehydfunktion, die zu größerer Kompatibilität mit anderen funktionellen Gruppen führt. Wie J.E. McMurry (Chem. Rev. 89, 1513 (1989), insbesondere Tabelle 2, S. 1515) beschreibt, ist das McMurry-Reagenz nur semikompatibel mit funktionellen Gruppen wie zum Beispiel Amid, Carbonsäure, Ester, Keton und inkompatibel mit funktionellen Gruppen wie Nitro, Oxim, Sulfoxid, Epoxid und 1,2-Diol. Hingegen ist der Vanadiumkomplex zum Beispiel vollständig kompatibel mit der Amidfunktion und selbst nicht aktivierte Aldehydfunktionen reagieren nicht mit nennenswerter Geschwindigkeit (J.H. Freudenberger, s.o., insbesondere S. 8016).

Verwendete Abkürzungen:
- Cha: Cyclohexylalanin
- Chg: Cyclohexylglycin
- DABCO: 1,4-Diazabicyclo[2.2.2]octan
- DMF: Dimethylformamid
- DMSO: Dimethylsulfoxid
- EDTA: Ethylendiamintetraessigsäure
- HMPA: Hexamethylphosphorsäuretriamid
- MTB: Methyl-tert. butyl
- Nal: 1- und 2-Naphthylalanin
- Npg: Neopentylglycin
- Tbg: Tert. Butylglycin
- THF: Tetrahydrofuran
- Thia: 2-Thienylalanin
- TMEDA: N, N,N',N'-Tetramethylethylendiamin
Durch die folgenden Beispiele wird das erfindungsgemäße Verfahren weiter illustriert und es werden konkrete Durchführungsvarianten geschildert. Diese Beispiele und Durchführungsvarianten limitieren den Erfindungsgegenstand weder bezüglich der strukturellen Bandbreite der auf diese Weise diastereoselektiv reduktiv dimerisierten α-Aminoaldehyde VII, noch bezüglich der Verfahrensbedingungen, (Reagenzherstellung, physikalische Parameter der reduktiven Dimerisierung, Reaktionszeit, Aufarbeitung, Reinigung und Analytik der Reaktionsprodukte).

### Beispiel 1 :

### N-(tert.-Butoxycarbonylamino)-(S)-Phenylalanin-N-methoxy-N-methylamid

88,1 g (332 mmol) (S)-Phenylalanin werden in 1,2 l Dichlormethan gelöst und unter Stickstoffatmosphäre bei einer konstanten Innentemperatur von 20 °C (Kühlung durch Eisbad) mit 268 ml (2,1 mol) Ethylmorpholin und 43,7 g (445 mmol) N,O-Dimethylhydroxylamin Hydrochlorid versetzt. Das Reaktionsgemisch wird auf -10 °C abgekühlt und eine Lösung von 252 ml Propanphosphonsäureanhydrid in 250 ml Essigsäureethylester zugetropft. Durch Nachrühren (1 h bei 0 °C, anschließend 2 h bei Raumtemperatur) wird vollständiger Umsatz der Reaktanden erreicht. Man wäscht mit 1 l 3 N HCl, 800 ml gesättigter, wäßriger NaHCO₃-Lösung, 800 ml gesättigter wässriger NaCl-Lösung und trocknet die organische Phase über Na₂SO₄. Nach dem Verdampfen des Lösungsmittels verbleiben 100 g eines farblosen Öles, das ohne weitere Reinigung der Reduktion zum entsprechenden Aminoaldehyd zugeführt werden kann.

### Beispiel 2 :

### N-(tert.-Butoxycarbonyl)-(S)-Phenylalaninal

4,36 g Lithiumaluminiumhydrid werden in 875 ml trockenem Diethylether unter Stickstoffatmosphäre bei 0 °C vorgelegt und unter Rühren eine Lösung von 26,9 g N-(tert.-Butoxycarbonylamino)-(S)-Phenylalanin-N-methoxy-N-methylamid in 73 ml Diethylether zugegeben. Es wird 30 min bei 0 °C gerührt und danach mit 450 ml 5%iger kalter wäßriger KHSO₄-Lösung versetzt. Die Phasen werden getrennt, die organische Phase nacheinander mit 300 ml 0,5 N HCl, 600 ml gesättigter wäßriger NaHCO₃-Lösung, 600 ml gesättigter wäßriger NaCl-Lösung gewaschen und schließlich über Na₂SO₄ getrocknet. Nach dem Verdampfen des Lösungsmittels verbleiben 20,9 g (96,3 %) weiße Kristalle, die ohne weitere Aufreinigung zur reduktiven Kupplung eingesetzt werden können.

### Beispiel 3 :

### (N-(tert.-Butoxycarbonylvalinyl)amino)-(S)-Phenylalaninal

2,1 ml (25 mmol) Oxalylchlorid werden unter Inertgasatmosphäre in 125 ml trockenem Dichlormethan gelöst. Bei -70 °C tropft man unter Rühren 2,4 ml (33,4 mmol) DMSO ein und fügt nach 15 min Wartezeit eine Lösung von 5,85 g (16,7 mmol) N-(tert.-Butoxycarbonyl)-(S)-Valyl-(S)-Phenylalaninol in einer Mischung aus 4 ml DMSO und 30 ml Dichlormethan langsam zu. Das Reaktionsgemisch wird 30 min bei -70 °C nachgerührt und dann 9,4 ml (66,8 mmol) Triethylamin eingetropft, wobei die Temperatur auf -60 °C ansteigt. Nach weiteren 15 min bei -60 °C hydrolysiert man mit 200 ml 15% iger wäßriger Zitronensäure und trennt die Phasen. Die organische Phase wird nacheinander mit jeweils 200 ml gesättigter wäßriger Bicarbonatlösung, mit Wasser, schließlich mit gesättigter wäßriger Kochsalzlösung gewaschen und über Natriumsulfat getrocknet. Nach dem Verdampfen des Lösungsmittels verbleiben 4,4 g weiße Kristalle die ohne weitere Reinigungschritte der Dimerisierung zugeführt werden.

### Versuchsvorschrift zur reduktiven Kupplung unter in situ - Darstellung des Kupplungsreagenzes

2.01 mmol Vanadiumtrichlorid werden 5 h unter Inertgasatmosphäre in 4,5 ml absolutem THF am Rückfluß gekocht. Nach dem Abkühlen auf RT gibt man 1,3 mmol Zinkstaub zu, rührt 30 min, versetzt mit 5,6 mmol Komplexbildner und erhitzt erneut zum Rückfluß. Nach Erreichen der Rückflußtemperatur werden 2 mmol des entsprechenden Aldehyds in 1 ml trockenem THF rasch zugegeben. Der vollständige Umsatz der Reaktanden wird mittels DC-Kontrolle überprüft. Nach Beendigung der Reaktion liefert Ausschütteln bei Raumtemperatur mit 1.) Natriumtartratlösung und 2.) kalter Zitronensäure (jeweils 20 ml einer 10% igen wäßrigen Lösung), Trocknen der organischen Phase über Na₂SO₄ und Absaugen des Lösungsmittels einen farblosen, öligen Rückstand, der durch Chromatographie an Kieselgel und/oder durch Kristallisation gereinigt wird.

### Beispiel 4 :

### 1,2-Bis[N-(tert.-Butoxycarbonyl)-(S)-1,2,3,4-tetrahydroisochinolin-3-yl]ethan-1(R),2(R)-diol

Ausgehend von 0,8 g N-(tert.-Butoxycarbonyl)-1,2,3,4-tetrahydroisochinolin-3(S)-carbaldehyd erhält man 0,28 g farblose Kristalle der Titelverbindung analog der Versuchsvorschrift unter in situ - Darstellung des Kupplungsreagenzes (Komplexbildner: 1,3-Dimethylimidazolidin-2-on).
- Smp.:: 203 °C
- MS (FAB):: 525 (M+H⁺), 415, 325

### Beispiel 5 :

### (25,3R,4R,5S)-2,5-(N,N'-(Benzyloxycarbonyl-(S)-valinyl)amino)-3,4-dihydroxy-1,6-diphenylhexan

Ausgehend von 764 mg N-Benzyloxycarbonyl-(S)-valinyl-phenylalaninal erhält man analog der Versuchsvorschrift unter in situ - Darstellung des Kupplungsreagenzes 420 mg weiße Kristalle der Titelverbindung (Komplexbildner: 1,3-Dimethylimidazolidin-2-on)
- Smp.:: 201 °C
- MS (FAB):: 767 (M + H⁺), 497

### Beispiel 6 :

### 1,2-Bis[N[{2(S)-(1,1-dimethylethylsulfonylmethyl)-3-(1-naphthyl)-propionyl}-(S)-valyl]-(S)-1,2,3,4-tetrahydroisochinolin-3-yl]-ethan-1(R),2(R)-diol

Ausgehend von 1,5 g N[{(S)-2-(1,1-Dimethylethylsulfonylmethyl)-3-(1-naphthyl)propionyl}-(S)-valyl]-(S)-1,2,3,4-tetrahydroisochinolin-3-carbaldehyd erhält man 0,3 g gelbliche Kristalle der Titelverbindung analog der Versuchsvorschrift unter in situ - Darstellung des Kupplungsreagenzes (Komplexbildner: 1,3-Dimethylimidazolidin-2-on).
- Smp.:: 152 °C (Zers.)
- MS (FAB):: 1162 (M+Li⁺), 1156 (M+H⁺), 741, 388

### Beispiel 7 :

### N,N'-Bis-(tert.-Butoxycarbonyl)-2S,5S-diamino-1,6-diphenyl-hexan-3R,4R-diol

Ausgehend von 5 g N-(tert.-Butoxycarbonyl)-(S)-phenylalaninal erhält man 3,4 weiße Kristalle der Titelverbindung analog der Versuchsvorschritt unter in situ-Darstellung des Kupplungsreagenzes (Komplexbildner: 1,3-Dimethylimidazolidin-2-on).
- Smp.:: 200 °C
- MS (FAB):: 501 (M+H⁺), 401, 345, 301

### Beispiel 8 :

### N, N'-Bis[{(S)-2-(1,1-dimethylethylsulfonylmethyl)-3-(1-naphthyl)-propionyl}-(S)-valyl]-2(S),5(S)-diamino-1,6-diphenylhexan-3(R),4(R)-diol

Ausgehend von 30,0 g [{ (S)-2-(1,1-Dimethylethylsulfonylmethyl)-3-(1-naphthyl)propionyl}-(S)-valyl]-(S)-phenylalaninal erhält man 16,5 g farblose Kristalle der Titelverbindung analog der Versuchsvorschrift unter in situ - Darstellung des Kupplungsreagenzes (Komplexbildner: 1,3-Dimethylimidazolidin-2-on).
- Smp.:: 187 °C
- MS (FAB):: 1153 (M+Na⁺), 1131 (M+H⁺), 716, 416

### Beispiel 9 (vgl. Beispiel 10) :

### (2S,3R,4R,5S)-2,5-Bis-[N-(tert-butoxycarbonyl)amino]-1,6-diphenyl-3,4-hexandiol

Die Suspension von 6,2 g Vanadiumtrichlorid in 165 ml trockenem THF wird mit Argon entgast und dann 5 Stunden in einer Argon-Atmosphäre unter Rückfluß gekocht. Man kühlt auf Raumtemperatur und gibt 1,53 g Zinkpulver zu. Man rührt 30 Min und gibt 14,6 ml 1,3-Dimethylimidazolidin-2-on per Spritze zu. Man erhitzt unter Argon zum Rückfluß und gibt die Lösung von 4,6 g N-(tert-Butoxycarbonyl)-(S)-phenylalaninal in 20 ml trockenem THF auf einmal zu. Das Gemisch wird 2 Std. unter Argon unter Rückfluß gekocht. DC (Aceton / Dichlormethan 1 : 3 ) zeigt unvollständige Reaktion nach 1 Std. und vollständige Reaktion nach 2 Std. an. Man läßt das Gemisch über Nacht bei Raumtemperatur stehen. 500 ml Ethylacetat wird zugegeben und die Lösung mit 2 x 250 ml 15% iger Zitronensäurelösung und mit 2 x 250 ml Wasser gewaschen. Die organische Phase wird über Magnesiumsulfat getrocknet und die Lösungsmittel im Vak. entfernt. Man erhält 3,97 g (86%) eines blaßgelben Feststoffs, 40% des (S,R,R,S)-lsomeren (HPLC) enthaltend.

### Vergleichsbeispiel

(2S,3R,4R,5S)- 2,5-Bis-[N-(tert-butoxycarbonyl)amino]-1,6-diphenyl-3,4-hexandiol Versuch der Herstellung durch Kupplung in Abwesenheit eines Komplexbildners bei 22°C Die Suspension von 3,1 g Vanadiumtrichlorid in 85 ml trockenem THF wird mit Argon entgast und dann 5 Std. in einer Argon-Atmosphäre unter Rückfluß gekocht. Man kühlt auf Raumtemperatur und gibt 760 mg Zinkpulver zu. Das Gemisch wird 0,5 Std. gerührt. Eine Lösung von 2,8 g N-(tert-butoxycarbonyl)-(S)-phenylalaninal in 30 ml trockenem THF wird bei 22°C zugegeben und das Gemisch 3 Std. bei Raumtemperatur gerührt. DC zeigt an, daß keine Kupplungsreaktion staflfand. 7,3 ml 1,3-Dimethylimidazolin-2-on werden zugegeben und weiter bei 22°C gerührt. Nach 3 Std. liegt gemäß DC unvollständige, nach 16 Std. quantitative Umwandlung des Edukts zum Kupplungsprodukt vor.

### Beispiel 10 (vgl. Beispiel 9)

### (2S,3R,4R,SS)-2,5-Bis-[N-(tert-butoxycarbonyl)amino]-1,6-diphenyl-3,4-hexandiol hergestellt durch Kupplung in Gegenwart eines Komplexbildners bei 22°C

Die Reaktion wird genau wie in dem Vergleichsbeispiel beschrieben durchgeführt, aber 7,3 ml 1,3-Dimethylimidazolin-2-on werden unmittelbar vor der Zugabe der Aldehyd-Lösung zugegeben. Dabei wird der Edukt-Aldehyd bei 22°C ins Titelprodukt umgewandelt. Die Reaktion ist nach 4 Std. weitgehend vollständig und nach 16 Std. quantitativ.

## Patentansprüche

1. Verfahren zur Herstellung von Verbindungen der Formel I 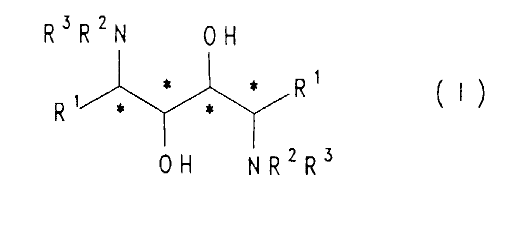 worin
R¹ für eine Seitenkettenrest einer natürlichen oder nicht-natürlichen α-Aminosäure steht;
R² und R³ gleich oder verschieden sind und
a)
- Wasserstoff
b)
- einen Rest der Formel
D-(E)ₙ-(F)ₒ-(G)ₚ (II)
bedeuten, wobei E, F und G unabhängig voneinander für eine natürliche oder unnatürliche Aminosäure, Azaaminosäure oder Iminosäure stehen;
n, o, p unabhängig voneinander 0 oder 1 bedeuten;
D für R⁴ oder einen Rest der Formeln III, IV oder V steht 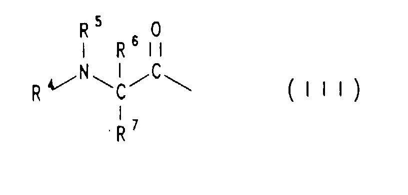 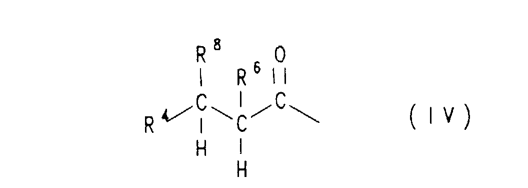 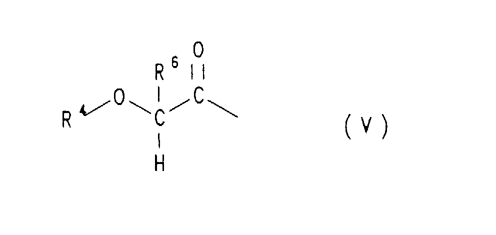 worin R⁴ bedeutet
b₁₎
- Wasserstoff,
- Carboxyl,
- (C₁-C₁₈)-Alkyl, das gegebenenfalls einfach oder zweifach ungesättigt ist und das gegebenenfalls durch bis zu 3 gleiche oder verschiedene Reste aus der Reihe
- Mercapto,
- Hydroxy,
- (C₁-C₇)-Alkoxy,
- Carbamoyl,
- (C₁-C₈)-Alkanoyloxy,
- Carboxy,
- (C₁-C₇)-Alkoxycarbonyl,
- F, Cl, Br, I,
- Amino,
- Amidino, das gegebenenfalls durch einen, zwei oder drei (C₁-C₈)-Alkylreste substituiert sein kann,
- Guanidino, das gegebenenfalls durch einen oder zwei Benzyloxycarbonylreste oder durch einen, zwei, drei oder vier (C₁-C₈)-Alkylreste substituiert sein kann,
- (C₁-C₇)-Alkylamino,
- Di-(C₁-C₇)-Alkylamino,
- (C₁-C₆)-Alkoxycarbonylamino,
- (C₇-C₁₅)-Aralkoxycarbonyl,
- (C₇-C₁₅)-Aralkoxycarbonylamino,
- Phenyl-(C₁-C₄)-alkoxy,
- 9-Fluorenylmethoxycarbonylamino,
- (C₁-C₆)-Alkylsulfonyl,
- (C₁-C₆)-Alkylsulfinyl,
- (C₁-C₆)-Alkylthio,
- Hydroxamino,
- Hydroximino,
- Sulfamoyl,
- Sulfo,
- Carboxamido,
- Formyl,
- Hydrazono,
- Imino,
- einen Rest CONR⁹R¹⁰,
- durch bis zu sechs Hydroxy oder
- durch bis zu fünf (C₁-C₈)-Alkanoyloxy substituiert ist;
- mono-, bi- oder tricyclisches (C₃-C₁₈)-Cycloalkyl,
- (C₃-C₁₈)-Cycloalkyl-(C₁-C₆)-alkyl, wobei der Cycloalkylteil jeweils gegebenenfalls durch einen oder zwei gleiche oder verschiedene Reste aus der Reihe
- F, Cl, Br, I,
- Carboxy,
- Carbamoyl,
- Carboxymethoxy,
- Hydroxy,
- (C₁-C₇)-Alkoxy,
- (C₁-C₇)-Alkyl
- (C₁-C₇)-Alkyloxycarbonyl,
- Amino,
- (C₁-C₆)-Alkylamino-(C₁-C₆)-alkyl,
- Di-(C₁-C₆)-Alkylamino-(C₁-C₆)-alkyl,
- Amidino,
- Hydroxamino,
- Hydroximino,
- Hydrazono,
- Imino,
- Guanidino,
- (C₁-C₆)-Alkoxysulfonyl,
- (C₁-C₆)-Alkoxysulfinyl,
- (C₁-C₆)-Alkoxycarbonylamino,
- (C₆-C₁₂)-Aryl-(C₁-C₄)-alkoxycarbonylamino,
- (C₁-C₇)-Alkylamino,
- Di-(C₁-C₇)-alkylamino und
- Trifluormethyl substituiert ist;
- (C₆-C₁₄)-Aryl,
- (C₆-C₁₄)-Aryl-(C₁-C₆)-alkyl,
- (C₆-C₁₄)-Aryloxy-(C₁-C₆)-alkyl oder
- (C₆-C₁₄)-Aryl-(C₃-C₈)-cycloalkyl, worin der Arylteil jeweils gegebenenfalls durch einen, zwei oder drei gleiche oder verschiedene Reste aus der Reihe
- F, Cl, Br, I,
- Hydroxy,
- Mono-, Di- oder Trihydroxy-(C₁-C₄)-alkyl,
- Trifluormethyl,
- Formyl,
- Carboxamido,
- Mono- oder Di- (C₁-C₄)-alkylaminocarbonyl,
- Nitro,
- (C₁-C₇)-Alkoxy,
- (C₁-C₇)-Alkyl,
- (C₁-C₇)-Alkoxycarbonyl,
- Amino,
- (C₁-C₇)-Alkylamino,
- Di-(C₁-C₇)-alkylamino,
- Carboxy,
- Carboxymethoxy,
- Amino-(C₁-C₇)-alkyl,
- (C₁-C₇)-Alkylamino-(C₁-C₇)-alkyl,
- Di-(C₁-C₇)-alkylamino-(C₁-C₇)-alkyl,
- (C₁-C₇)-Alkoxycarbonylmethoxy,
- Carbamoyl,
- Sulfamoyl,
- (C₁-C₇)-Alkoxysulfonyl,
- (C₁-C₈)-Alkylsulfonyl,
- Sulfo-(C₁-C₈)-alkyl,
- Guanidino-(C₁-C₈)-alkyl und
- (C₁-C₆)-Alkoxycarbonylamino substituiert ist;
- Het,
- Het-(C₁-C₆)-alkyl,
- Het-(C₃-C₈)-cycloalkyl,
- Het-(C₃-C₈)-cycloalkyl-(C₁-C₄)-alkyl,
- Het-(C₃-C₈)-cycloalkoxy-(C₁-C₄)-alkyl,
- Het-thio-(C₁-C₆)-alkyl,
- Het-thio-(C₃-C₈)-cycloalkyl,
- Het-thio-(C₃-C₈)-cycloalkyl-(C₁-C₄)-alkyl, wobei Het jeweils für den Rest eines 5- bis 7-gliedrigen monocyclischen oder 8- bis 10-gliedrigen bicyclischen Ringsystems steht, das benzanelliert, aromatisch, teil- oder vollständig hydriert sein kann, das als Heteroelemente einen, zwei, drei oder vier verschiedene Reste aus der Gruppe N, O, S, NO, SO, SO₂ enthalten kann, das mit 1 bis 6 Hydroxy substituiert sein kann und das gegebenenfalls wie bei (C₆-C₁₄)-Aryl unter b₁) definiert und/oder mit Oxo mono-, di- oder trisubstituiert ist, oder einen Rest NR⁹R¹⁰ bedeutet oder,
b₂)
- einen Rest der Formel VI
R^{4a} - W (VI)
worin R^{4a} wie R⁴ unter b₁) definiert ist und W für -CO-, -CS-, O-CO-, -SO₂-, -SO-, -S-, -NHSO₂-, -NHCO-, -CH(OH)-, -N(OH)- oder -CO-V-, wobei V ein Peptid mit insgesamt 1 bis 10 Amino-, Imino-und/oder Azaaminosäuren bedeutet, steht; oder worin R⁴ zusammen mit R⁸ und den diese tragenden Atomen mono- oder bicyclische, gesättigte oder teilweise ungesättigte Ringsysteme mit 5-12 Ringgliedern bilden, die außer Kohlenstoff noch 1 Schwefelatom enthalten können, welches gegebenenfalls zum Sulfoxid oder Sulfon oxidiert sein kann;
b₃)
- einen Glycosylrest, bevorzugt einen Glucofuranosyl- oder Glucopyranosyl-Rest, der sich von natürlich vorkommenden Aldotetrosen, Aldopentosen, Aldohexosen, Ketopentosen, Ketohexosen, Desoxyaldosen, Aminoaldosen und Oligosacchariden sowie deren Stereoisomeren ableitet; oder
b₄)
- eine Aminoschutzgruppe;
R⁵
- Wasserstoff oder
- (C₁-C₈)-Alkyl bedeutet, oder
- zusammen mit R⁶ und den diesen Rest tragenden Atomen mono-oder bicyclische, gesättigte oder teilweise ungesättigte Ringsysteme mit 5-12 Ringgliedern bildet;
R⁶
- wie R⁴ unter b₁) definiert ist;
- für Hydroxy oder (C₁-C₄)-Alkanoyloxy steht; oder
- zusammen mit R⁷ und den diesen Rest tragenden Atomen cyclische, gesättigte oder teilweise ungesättigte Ringsysteme mit 3 bis 12 Ringgliedern bildet; oder
- zusammen mit R⁸ und den diese tragenden Atomen ein mono-oder bicyclisches, gesättigtes oder teilweise ungesättigtes Ringsystem mit 5-12 Ringgliedern bildet, das außer Kohlenstoff noch 1 Schwefelatom enthalten kann, welches gegebenenfalls zum Sulfoxid oder Sulfon oxidiert sein kann; oder 1 Stickstoffatom enthalten kann, wobei das Ringsystem gegebenenfalls durch Amino substituiert sein kann;
R⁷
- Wasserstoff oder
- (C₁-C₆)-Alkyl bedeutet;
R⁸
- Wasserstoff,
- Hydroxy,
- (C₁-C₄)-Alkanoyloxy oder
- (C₁-C₈)-Alkyl bedeutet;
R⁹ und R¹⁰
- Wasserstoff,
- (C₁-C₈)-Alkyl, das durch
- Amino,
- (C₁-C₄)-Alkylamino,
- Di-(C₁-C₄)-alkylamino,
- Mercapto,
- Carboxy,
- Hydroxy oder
- (C₁-C₄)-Alkoxy substituiert sein kann,
- (C₃-C₇)-Cycloalkyl,
- (C₁-C₄)-Alkoxycarbonyl,
- (C₆-C₁₄)-Aryl, (C₆-C₁₄)-Aryl-(C₁-C₄)-alkoxycarbonyl, die im Arylteil wie bei R⁴ beschrieben, substituiert sein können,
- Het oder
- Het-(C₁-C₄)-alkyl, wobei Het wie bei R⁴ beschrieben definiert ist, bedeuten oder wobei
R⁹ und R¹⁰ zusammen mit dem sie tragenden Stickstoffatom monocyclische oder bicyclische, gesättigte, teilweise ungesättigte oder aromatische Ringsysteme bilden, die als Ringglieder neben Kohlenstoff noch 1 oder weitere 2 Stickstoffatome, 1 Schwefelatom oder 1 Sauerstoffatom enthalten und durch (C₁-C₄)-Alkyl substituiert sein können, wobei in den vorstehenden Verbindungen der Formel I eine oder mehrere Amidgruppen (-CONH-) der Hauptkette ersetzt sein können durch -CH₂NR¹¹-, -CH₂S-, -CH₂O-, -OCH₂-, -CH₂CH₂-, -CH=CH-(cis und trans), -COCH₂-, -CH(OH)CH₂-, -CH₂SO-, -CH₂SO₂-, -COO-, -P(O)(OR¹²)CH₂- und -P(O)(OR¹²)NH-, oder auch durch eine Amidgruppe mit umgekehrter Polarität (-NHCO-); worin R¹¹ und R¹² unabhängig voneinander stehen für
- Wasserstoff oder
- (C₁-C₄)-Alkyl;
sowie deren Enantiomere und physiologisch verträgliche Salze, dadurch gekennzeichnet, daß homochirale α-Aminoaldehyde der Formel VII 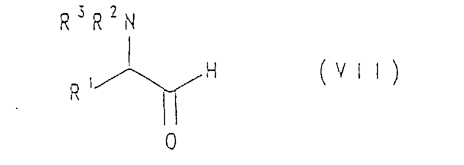 worin R¹, R², und R³ wie oben definiert sind, mit einem in situ aus VCl₃, THF und Zinkstaub erhaltenen VanadiumKomplex in THF und unter Zusatz eines Komplexbildners behandelt werden, wobei eine gleichzeitige Kontrolle über alle vier Chiralitätszentren vorliegt.

2. Verfahren zur Herstellung von Verbindungen der Formel I gemäß Anspruch 1, dadurch gekennzeichnet, daß
R¹ einen Seitenkettenrest der α-Aminosäuren Gly, Ala, Val, Leu, lle, Ser, Thr, Cys, Met, Pro, Lys, Arg, His, Asp, Asn, Glu, Gln, Phe, Tyr, Trp oder Cha bedeutet;
R² und R³ gleich oder verschieden sind und
a)
- Wasserstoff
b)
- einen Rest der Formel II
worin o und p = 0,
n = 0 oder 1 ist und
E für eine der oben genannten α-Aminosäuren steht,
D für R⁴ oder einen Rest der Formeln III oder IV steht, worin R⁴
b₁₎
- Wasserstoff
- (C₁-C₉)-Alkyl, das gegebenenfalls einfach oder zweifach ungesättigt ist und das gegebenenfalls durch bis zu 3 gleiche oder verschiedene Reste aus der Reihe
- Hydroxy,
- (C₁-C₇)-Alkoxy,
- Carbamoyl,
- (C₁-C₈)-Alkanoyloxy,
- (C₁-C₇)-Alkoxycarbonyl,
- F, Cl,
- Amino,
- (C₁-C₇)-Alkylamino,
- Di-(C₁-C₇)-Alkylamino,
- (C₁-C₆)-Alkoxycarbonylamino,
- (C₇-C₁₅)-Aralkoxycarbonyl,
- (C₇-C₁₅)-Aralkoxycarbonylamino,
- Phenyl-(C₁-C₄)-alkoxy,
- 9-Fluorenylmethoxycarbonylamino,
- (C₁-C₆)-Alkylsulfonyl,
- (C₁-C₆)-Alkylsulfinyl,
- (C₁-C₆)-Alkylthio subsitutiert ist,
- (C₆-C₁₄)-Aryl,
- (C₆-C₁₄)-Aryl-(C₁-C₆)-alkyl oder
- (C₆-C₁₄)-Aryloxy-(C₁-C₆)-alkyl, worin der Arylteil jeweils gegebenenfalls durch einen, zwei oder drei gleiche oder verschiedene Reste aus der Reihe der oben genannten bevorzugten Substituenten von (C₁-C₉)-Alkyl substituiert sein kann,
b₂₎
- einen Rest der Formel VI, worin R^{4a} wie R⁴ unter b₁₎ definiert ist und W für -CO-, O-CO-, -SO₂-, -SO-, -S-, -NHCO-, -CH(OH)- steht;
b₄₎
- eine Aminoschutzgruppe Fmoc, Z oder Boc bedeutet,
R⁵ und R⁷ Wasserstoff bedeuten,
R⁶
- wie R⁴ definiert ist, und
R⁸
- Wasserstoff,
- Hydroxy,
- (C₁-C₄)-Alkanoyloxy oder
- (C₁-C₈)-Alkyl bedeutet.

3. Verfahren zur Herstellung von Verbindungen der Formel I gemäß einem der Ansprüche 1 oder 2, dadurch gekennzeichnet, daß einer der Reste R² oder R³ Wasserstoff bedeutet.

4. Verfahren zur Herstellung von Verbindungen der Formel I gemäß einem der Ansprüche 1 bis 3, dadurch gekennzeichnet, daß die Verbindungen der Formel I SRRS-Konfiguration oder RSSR-Konfiguration haben.

5. Verfahren zur Herstellung von Verbindungen der Formel I gemäß einem der Ansprüche 1 bis 4, dadurch gekennzeichnet, daß
R¹
- einen Seitenkettenrest der α-Aminosäuren Ala, Val, Leu, Ile, Pro, Phe, Cha oder Tyr bedeutet,
R² und R³ gleich oder verschieden sind und
a)
- Wasserstoff
b)
- einen Rest der Formel II, worin
o und p = 0 sind,
n 0 oder 1 bedeutet und
E für Ala, Val, Leu, Ile, Pro, Phe, Cha oder Tyr steht;
D für R⁴ oder einen Rest der Formel IV steht mit R⁴
b₁₎
- Wasserstoff,
- (C₁-C₄)-Alkyl,
- Phenyl oder Naphthyl,
- Phenylmethyl- oder Naphthylmethyl,
b₂)
- Rest der Formel VI, worin R^{4a} wie R⁴ unter b₁₎ definiert ist und W für -CO-, -O-CO-, -SO₂-, -SO-, -S- , -NHCO-, -CH(OH)- steht, oder
b₄)
- eine Aminoschutzgruppe Fmoc, Z oder Boc bedeuten,
R⁵, R⁷ und R⁸ Wasserstoff bedeuten, und
R⁶ wie R⁴ unter b₁) definiert ist.

6. Verfahren zur Herstellung von Verbindungen der Formel I gemäß den Ansprüchen 1 bis 5, dadurch gekennzeichnet, daß man VCl₃ in einer schutzgasgespülten Apparatur in THF bei Temperaturen von -78 °C bis Siedetemperatur vorlegt und sukzessive mit 0,5 bis 1,0 Equivalenten Zinkstaub und bis 9 Equivalenten eines Komplexbildners und mit 0,2 bis 1,0 Equivalenten Aldehyd der allgemeinen Formel VII versetzt und bis zur Reaktionsvervollständigung unter Schutzgasatmosphäre bei der jeweiligen Anfangstemperatur rührt.

## Claims

1. A process for the preparation of a compound of the formula I 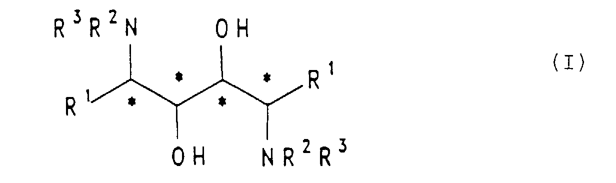 in which
R¹ is a side chain radical of a natural or synthetic α-amino acid;
R² and R³ are identical or different and are each
a) - hydrogen
b) - a radical of the formula
D-(E)ₙ-(F)ₒ-(G)ₚ (II)
where E, F and G independently of each other are a natural or synthetic amino acid, azaamino acid or imino acid;
n, o and p independently of each other are 0 or 1;
D is R⁴ or a radical of the formula III, IV or V 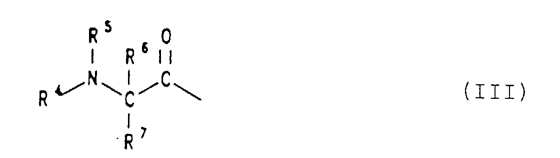 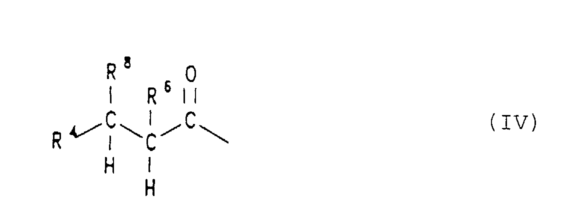 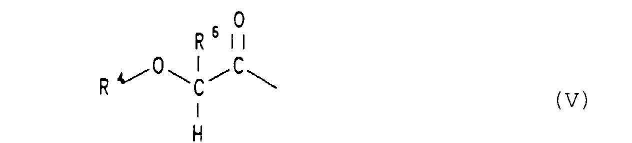 in which R⁴ is
b₁)
- hydrogen,
- carboxyl,
- (C₁-C₁₈)-alkyl, which is optionally monounsaturated or diunsaturated and which is unsubstituted or substituted by up to 3 identical or different radicals selected from the group comprising
- mercapto,
- hydroxyl,
- (C₁-C₇)-alkoxy,
- carbamoyl,
- (C₁-C₈) -alkanoyloxy,
- carboxyl,
- (C₁-C₇)-alkoxycarbonyl,
- F, Cl, Br, I,
- amino,
- amidino, which can be unsubstituted or substituted by one, two or three (C₁-C₈) -alkyl radicals,
- guanidino, which can be unsubstituted or substituted by one or two benzyloxycarbonyl radicals or by one, two, three or four (C₁-C₈)-alkyl radicals,
- (C₁-C₇) -alkylamino,
- di- (C₁-C₇) -alkylamino,
- (C₁-C₆)-alkoxycarbonylamino,
- (C₇-C₁₅) -aralkoxycarbonyl,
- (C₇-C₁₅) -aralkoxycarbonylamino,
- phenyl- (C₁-C₄) -alkoxy,
- 9-fluorenylmethoxycarbonylamino,
- (C₁-C₆) -alkylsulfonyl,
- (C₁-C₆) -alkylsulfinyl,
- (C₁-C₆) -alkylthio,
- hydroxamino,
- hydroximino,
- sulfamoyl,
- sulfo,
- carboxamido,
- formyl,
- hydrazono,
- imino,
- a CONR⁹R¹⁰ radical,
- by up to six hydroxyl groups or
- by up to five (C₁-C₈)-alkanoyloxy groups;
- mono-, bi- or tricyclic (C₃-C₁₈)-cycloalkyl,
- (C₃-C₁₃) -cycloalkyl-(C₁-C₆)-alkyl, the cycloalkyl moiety in each case being unsubstituted or substituted by one or two identical or different radicals selected from the group comprising
- F, Cl, Br, I,
- carboxyl,
- carbamoyl,
- carboxymethoxy,
- hydroxyl,
- (C₁-C₇)-alkoxy,
- (C₁-C₇)-alkyl
- (C₁-C₇) -alkyloxycarbonyl,
- amino,
- (C₁-C₆) -alkylamino- (C₁-C₆)-alkyl,
- di- (C₁-C₆) -alkylamino- (C₁-C₆) -alkyl,
- amidino,
- hydroxamino,
- hydroximino,
- hydrazono,
- imino,
- guanidino,
- (C₁-C₆) -alkoxysulfonyl,
- (C₁-C₆) -alkoxysulfinyl,
- (C₁-C₆) -alkoxycarbonylamino,
- (C₆-C₁₂) -aryl- (C₁-C₄)-alkoxycarbonylamino,
- (C₁-C₇) -alkylamino,
- di-(C₁-C₇) -alkylamino and
- trifluoromethyl;
- (C₆-C₁₄)-aryl,
- (C₆-C₁₄)-aryl-(C₁-C₆)-alkyl,
- (C₆-C₁₄)-aryloxy-(C₁-C₆)-alkyl or
- (C₆-C₁₄)-aryl-(C₃-C₈)-cycloalkyl, in which the aryl moiety in each case is unsubstituted or substituted by one, two or three identical or different radicals selected from the group comprising
- F, Cl, Br, I,
- hydroxyl,
- mono-, di- or trihydroxy-(C₁-C₄)-alkyl,
- trifluoromethyl,
- formyl,
- carboxamido,
- mono- or di- (C₁-C₄)-alkylaminocarbonyl,
- nitro,
- (C₁-C₇)-alkoxy,
- (C₁-C₇)-alkyl,
- (C₁-C₇) -alkoxycarbonyl,
- amino,
- (C₁-C₇)-alkylamino.
- di- (C₁-C₇) -alkylamino,
- carboxyl,
- carboxymethoxy,
- amino- (C₁-C₇) -alkyl,
- (C₁-C₇)-alkylamino-(C₁-C₇)-alkyl,
- di- (C₁-C₇) -alkylamino- (C₁-C₇) -alkyl,
- (C₁-C₇) -alkoxycarbonylmethoxy,
- carbamoyl,
- sulfamoyl,
- (C₁-C₇) -alkoxysulfonyl,
- (C₁-C₈) -alkylsulfonyl,
- sulfo- (C₁-C₈) -alkyl,
- guanidino- (C₁-C₈) -alkyl and
- (C₁-C₆) -alkoxycarbonylamino;
- het,
- het-(C₁-C₆)-alkyl,
- het- (C₃-C₈) -cycloalkyl,
- het-(C₃-C₈)-cycloalkyl-(C₁-C₄)-alkyl,
- het -(C₃-C₈)-cycloalkoxy- (C₁-C₄)-alkyl,
- het-thio-(C₁-C₆)-alkyl,
- het-thio- (C₃-C₈) -cycloalkyl,
- het-thio-(C₃-C₈)-cycloalkyl-(C₁-C₄)-alkyl, het being in each case the radical of a 5- to 7-membered monocyclic or 8- to 10-membered bicyclic ring system, which may be fused to a benzene ring, be aromatic, or partially or completely hydrogenated, which can contain as hetero elements one, two, three or four different radicals selected from the group comprising N, O, S, NO, SO and SO₂, which can be substituted by 1 to 6 hydroxyl groups and which is optionally defined as for (C₆-C₁₄)-aryl under b₁) and/or is mono-, di- or trisubstituted by oxo, or is an NR⁹R¹⁰ radical, or
b₂)
- a radical of the formula VI
R^{4a} - W (VI)
in which R^{4a} is defined as for R⁴ under b₁) and W is -CO-, -CS-, O-CO-, -SO₂-, -SO-, -S-, -NHSO₂-, -NHCO-, -CH(OH)-, -N(OH)- or -CO-V-, V being a peptide having a total of 1 to 10 amino acids, imino acids and/or azaamino acids; or in which R⁴ together with R⁸ and the atoms bearing these form mono- or bicyclic, saturated or partially unsaturated ring systems having 5-12 ring members, which can also contain, apart from carbon, 1 sulfur atom which can optionally be oxidized to the sulfoxide or sulfone;
b₃)
- a glycosyl radical, preferably a glucofuranosyl or glucopyranosyl radical, which is derived from naturally occurring aldotetroses, aldopentoses, aldohexoses, ketopentoses, ketohexoses, deoxyaldoses, aminoaldoses and oligosaccharides and their stereoisomers; or
b₄)
- an amino-protecting group;
R⁵
- is hydrogen or
- (C₁-C₈) -alkyl, or
- together with R⁶ and the atoms bearing this radical forms mono- or bicyclic, saturated or partially unsaturated ring systems having 5-12 ring members;
R⁶ - is defined as for R⁴ under b₁);
- is hydroxyl or (C₁-C₄)-alkanoyloxy; or
- together with R⁷ and the atoms bearing this radical forms cyclic, saturated or partially unsaturated ring systems having 3 to 12 ring members; or
- together with R⁸ and the atoms bearing this forms a mono- or bicyclic, saturated or partially unsaturated ring system having 5-12 ring members, which can also contain, apart from carbon, 1 sulfur atom which can optionally be oxidized to the sulfoxide or sulfone; or can contain 1 nitrogen atom, where the ring system can be unsubstituted or substituted by amino;
R⁷
- is hydrogen or
- (C₁-C₆)-alkyl;
R⁸
- is hydrogen,
- hydroxyl,
- (C₁-C₄)-alkanoyloxy or
- (C₁-C₈) -alkyl;
R⁹ and R¹⁰ are each
- hydrogen,
- (C₁-C₈) -alkyl, which can be substituted by
- amino,
- (C₁-C₄) -alkylamino,
- di- (C₁-C₄) -alkylamino,
- mercapto,
- carboxyl,
- hydroxyl or
- (C₁-C₄)-alkoxy,
- (C₃-C₇) -cycloalkyl,
- (C₁-C₄) -alkoxycarbonyl,
- (C₆-C₁₄)-aryl, (C₆-C₁₄)-aryl- (C₁-C₄)-alkoxycarbonyl, which can be substituted in the aryl moiety as described for R⁴,
- het or
- het-(C₁-C₄)-alkyl, het being defined as described for R⁴, or
R⁹ and R¹⁰ together with the nitrogen atom bearing them forming monocyclic or bicyclic, saturated, partially unsaturated or aromatic ring systems which contain as ring members, in addition to carbon, 1 or 2 further nitrogen atoms, 1 sulfur atom or 1 oxygen atom and can be substituted by (C₁-C₄) -alkyl, where in the preceding compounds of the formula I one or more amide groups (-CONH-) of the main chain can be replaced by -CH₂-NR¹¹-, -CH₂S-, -CH₂O-, -OCH₂-, -CH₂CH₂ -, -CH=CH-(cisand trans), -COCH₂-, -CH(OH)CH₂-, -CH₂SO-, -CH₂SO₂-, -COO-, -P(O)(OR¹²)CH₂- and -P(O)(OR¹²)NH-, or alternatively by an amide group having reversed polarity (-NHCO-); in which R¹¹ and R¹² independently of each other are
- hydrogen or
- (C₁-C₄)-alkyl;
and their enantiomers and physiologically tolerated salts, which comprises treating a homochiral α-aminoaldehyde of the formula VII 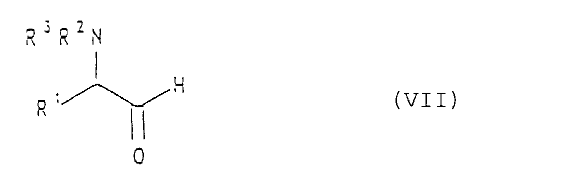 in which R¹, R² and R³ are defined as above, with a vanadium complex obtained in situ from VCl₃, THF and zinc dust in THF and with the addition of a complexing agent, a simultaneous control over all four chiral centers being present.

2. The process for the preparation of a compound of the formula I as claimed in claim 1, wherein
R¹ is a side chain radical of the α-amino acids Gly, Ala, Val, Leu, Ile, Ser, Thr, Cys, Met, Pro, Lys, Arg, His, Asp, Asn, Glu, Gln, Phe, Tyr, Trp or Cha;
R² and R³ are identical or different and are each
a)
- hydrogen
b)
- a radical of the formula II
in which o and p = 0,
n = 0 or 1 and
E is one of the abovementioned α-amino acids,
D is R⁴ or a radical of the formula III or IV, in which R⁴ is
b₁)
- hydrogen
- (C₁-C₉)-alkyl, which is optionally monounsaturated or diunsaturated and which is unsubstituted or substituted by up to 3 identical or different radicals selected from the group comprising
- hydroxyl,
- (C₁-C₇)-alkoxy,
- carbomoyl,
- (C₁-C₈)-alkanoyloxy,
- (C₁-C₇) -alkoxycarbonyl,
- F, Cl,
- amino,
- (C₁-C₇) -alkylamino,
- di- (C₁-C₇) -alkylamino,
- (C₁-C₆)-alkoxycarbonylamino,
- (C₇-C₁₅) -aralkoxycarbonyl,
- (C₇-C₁₅)-aralkoxycarbonylamino,
- phenyl- (C₁-C₄) -alkoxy,
- 9-fluorenylmethoxycarbonylamino,
- (C₁-C₆) -alkylsulfonyl,
- (C₁-C₆)-alkylsulfinyl, and
- (C₁-C₆)-alkylthio,
- (C₆-C₁₄)-aryl,
- (C₆-C₁₄)-aryl-(C₁-C₆)-alkyl or
- (C₆-C₁₄) -aryloxy- (C₁-C₆)-alkyl, in which the aryl moiety may in each case be unsubstituted or substituted by one, two or three identical or different radicals selected from the group comprising the abovementioned preferred substituents of (C₁-C₉)-alkyl,
b₂)
- a radical of the formula VI, in which R^{4a} is defined as for R⁴ under b₁) and W is -CO-, O-CO-, -SO₂-, -SO-, -S-, -NHCO-, -CH(OH)-;
b₄)
- an amino-protecting group Fmoc, Z or Boc,
R⁵ and R⁷ are each hydrogen,
R⁶
- is defined as for R⁴, and
R⁸
- is hydrogen,
- hydroxyl,
- (C₁-C₄)-alkanoyloxy or
- (C₁-C₈)-alkyl.

3. The process for the preparation of a compound of the formula I as claimed in one of claims 1 or 2, wherein one of the radicals R² and R³ is hydrogen.

4. The process for the preparation of a compound of the formula I as claimed in one of claims 1 to 3, wherein the compound of the formula I has the SRRS-configuration or the RSSR-configuration.

5. The process for the preparation of a compound of the formula I as claimed in one of claims 1 to 4, wherein
R¹ - is a side chain radical of the α-amino acids Ala, Val, Leu, Ile, Pro, Phe, Cha or Tyr,
R² and R³ are identical or different and are each
a) - hydrogen
b) - a radical of the formula II, in which
o and p = 0,
n is 0 or 1 and
E is Ala, Val, Leu, Ile, Pro, Phe, Cha or Tyr;
D is R⁴ or a radical of the formula IV where R⁴ is
b₁)
- hydrogen,
- (C₁-C₄)-alkyl,
- phenyl or naphthyl,
- phenylmethyl or naphthylmethyl,
b₂) - a radical of the formula VI, in which R^{4a} is defined as for R⁴ under b₁) and W is -CO-, -O-CO-, -SO₂-, -SO-, -S-, -NHCO-, -CH(OH)-, or
b₄) - an amino-protecting group Fmoc, Z or Boc,
R⁵, R⁷ and R⁸ are each hydrogen, and
R⁶ is defined as for R⁴ under b₁).

6. The process for the preparation of a compound of the formula I as claimed in any of claims 1 to 5, wherein VCl₃ is introduced into an apparatus flushed with a protecting gas in THF at temperatures from - 78°C to boiling point and 0.5 to 1.0 equivalent of zinc dust and up to 9 equivalents of a complexing agent and 0.2 to 1.0 equivalent of an aldehyde of the formula VII are successively added and the mixture is stirred under an atmosphere of protecting gas at the respective initial temperature until completion of the reaction.

## Revendications

1. Procédé pour la préparation de composés de formule I 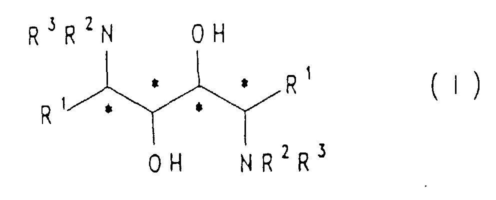 dans laquelle
R¹ représente un reste de chaîne latérale d'un α-aminoacide naturel ou non naturel;
R² et R³ sont identiques ou différents et représentent
a) un atome d'hydrogène,
b) un radical de formule
D-(E)ₙ-(F)ₒ-(G)ₚ (II)
E, F et G représentant, indépendamment les uns des autres, un aminoacide, azaaminoacide ou iminoacide naturel ou non naturel;
n, o, p représentant, indépendamment les uns des autres, 0 ou 1;
D représentant R⁴ ou un radical de formule III, IV ou V 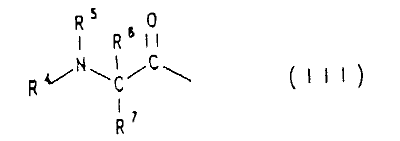 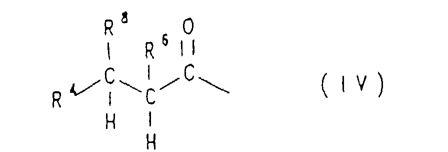 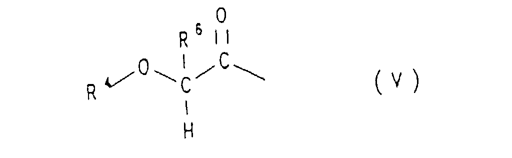 formules dans lesquelles
R⁴ représente
b₁)
- un atome d'hydrogène,
- le groupe carboxyle,
- un groupe alkyle en C₁-C₁₈ qui est éventuellement 1 ou 2 fois insaturé et qui peut éventuellement être substitué par jusqu'à 3 radicaux, identiques ou différents, choisis parmi les groupes
- mercapto,
- hydroxyle,
- alcoxy en C₁-C₇,
- carbamoyle,
- alcanoyloxy en C₁-C₈,
- carboxyle,
- alcoxy(C₁-C₇)carbonyle,
- F, Cl, Br, I,
- amino,
- amidino qui peut éventuellement être substitué par 1, 2 ou 3 radicaux alkyle en C₁-C₈,
- guanidino qui peut éventuellement être substitué par 1 ou 2 radicaux benzyloxycarbonyle ou par 1, 2, 3 ou 4 radicaux alkyle en C₁-C₈,
- alkyl(C₁-C₇)amino,
- dialkyl(C₁-C₇)amino,
- alcoxy(C₁-C₆) carbonylamino,
- aralcoxy(C₇-C₁₅) carbonyle,
- aralcoxy(C₇-C₁₅)carbonylamino,
- phényl-alcoxy(C₁-C₄),
- 9-fluorénylméthoxycarbonylamino,
- alkyl(C₁-C₆)sulfonyle,
- alkyl(C₁-C₆)sulfinyle,
- alkyl(C₁-C₆)thio,
- hydroxamino,
- hydroximino,
- sulfamoyle,
- sulfo,
- carboxamido,
- formyle,
- hydrazono,
- imino,
- un radical CONR⁹R¹⁰,
- par jusqu'à 6 groupes hydroxyle ou
- par jusqu'à 5 groupes alcanoyloxy en C₁-C₈;
- cycloalkyle en C₃-C₁₈ mono-, bi- ou tricyclique,
- cycloalkyl(C₃-C₁₈)-alkyle(C₁-C₆), le fragment cycloalkyle étant dans chaque cas éventuellement substitué par 1 ou 2 radicaux, identiques ou différents, choisis parmi
- F, Cl, Br, I,
- carboxyle,
- carbamoyle,
- carboxyméthoxy,
- hydroxyle,
- alcoxy en C₁-C₇,
- alkyle en C₁-C₇,
- alkyloxy(C₁-C₇)carbonyle,
- amino,
- alkyl(C₁-C₆)amino-alkyle(C₁-C₆),
- dialkyl(C₁-C₆)amino-alkyle(C₁-C₆),
- amidino,
- hydroxamino,
- hydroximino,
- hydrazono,
- imino,
- guanidino,
- alcoxy(C₁-C₆)sulfonyle,
- alcoxy(C₁-C₆)sulfinyle,
- alcoxy(C₁-C₆)carbonylamino,
- aryl(C₆-C₁₂)-alcoxy(C₁-C₄)carbonylamino,
- alkyl(C₁-C₇)amino,
- dialkyl(C₁-C₇)amino et
- trifluorométhyle;
- aryle en C₆-C₁₄,
- aryl(C₆-C₁₄)-alkyle(C₁-C₆),
- aryloxy(C₆-C₁₄)-alkyle(C₁-C₆) ou
- aryl(C₆-C₁₄)-cycloalkyle(C₃-C₈) le fragment aryle étant dans chaque cas éventuellement substitué par 1, 2 ou 3 radicaux, identiques ou différents, choisis parmi
- F, Cl, Br, I,
- hydroxyle,
- mono-, di- ou trihydroxyalkyle en C₁-C₄,
- trifluorométhyle,
- formyle,
- carboxamido,
- mono- ou dialkyl(C₁-C₄)aminocarbonyle,
- nitro,
- alcoxy en C₁-C₇,
- alkyle en C₁-C₇,
- alcoxy(C₁-C₇)carbonyle,
- amino,
- alkyl(C₁-C₇)amino,
- dialkyl(C₁-C₇)amino,
- carboxyle,
- carboxyméthoxy,
- aminoalkyle(C₁-C₇),
- alkyl(C₁-C₇)amino-alkyle(C₁-C₇),
- dialkyl(C₁-C₇)amino-alkyle(C₁-C₇),
- alcoxy(C₁-C₇)carbonylméthoxy,
- carbamoyle,
- sulfamoyle,
- alcoxy(C₁-C₇)sulfonyle,
- alkyl(C₁-C₈)sulfonyle,
- sulfoalkyle(C₁-C₈),
- guanidinoalkyle(C₁-C₈) et
- alcoxy(C₁-C₆)carbonylamino;
- Het,
- Het-alkyle(C₁-C₆),
- Het-cycloalkyle(C₃-C₈),
- Het-cycloalkyl(C₃-C₈)-alkyle(C₁-C₄),
- Het-cycloalcoxy(C₃-C₈)-alkyle(C₁-C₄),
- Het-thioalkyle(C₁-C₆),
- Het-thiocycloalkyle(C₃-C₈),
- Het-thiocycloalkyl(C₃-C₈)-alkyle(C₁-C₄), Het représentant dans chaque cas le reste d'un système cyclique monocyclique à 5-7 chaînons ou bicyclique à 8-10 chaînons, qui peut être aromatique, partiellement ou totalement hydrogéné ou condensé à un noyau benzénique, et qui peut contenir comme hétéroéléments 1, 2, 3 ou 4 atomes ou groupes différents choisis parmi N, O, S, NO, SO, SO₂, qui peut être substitué par 1 à 6 groupes hydroxyle et qui est éventuellement mono-, di- ou trisubstitué comme défini en b₁). pour le groupe aryle en C₆-C₁₄ et/ou par le groupe oxo, ou représente un radical NR⁹R¹⁰, ou
b₂) - un radical de formule VI
R^{4a}-W (VI)
dans laquelle R^{4a} est défini comme R⁴ en b₁), et W représente -CO-, -CS-, -O-CO-, SO₂-, -SO-, -S-, -NHSO₂-, -NHCO-, -CH(OH)-, -N(OH)- ou -CO-V-, V représentant un peptide comportant au total 1 à 10 amino-, imino- et/ou azaaminoacides; ou dans laquelle R⁴ forme, conjointement avec R⁸ et les atomes qui les portent, des systèmes cycliques mono- ou bicycliques, saturés ou partiellement insaturés, comportant 5-12 chaînons formant le(s) cycle(s), qui peuvent contenir, en plus d'atomes de carbone, encore un atome de soufre qui peut éventuellement être oxydé en sulfoxyde ou en sulfone;
b₃)- un radical glycosyle, de préférence un radical glucofurannosyle ou glucopyrannosyle, qui dérive d'aldotétroses, aldopentoses, aldohexoses, cétopentoses, cétohexoses, désoxyaldoses, amino-aldoses et oligosaccharides existant dans la nature, ainsi que de leurs stéréoisomères; ou
b₄) - un groupe protecteur de fonction amino;
R⁵ représente
- un atome d'hydrogène ou
- un groupe alkyle en C₁-C₈, ou
- conjointement avec R⁶ et les atomes qui les portent, des systèmes cycliques mono- ou bicycliques saturés ou partiellement insaturés, ayant de 5 à 12 chaînons formant le(s) cycle(s);
R⁶ - est défini comme R⁴ en b₁) ;
- représente un groupe hydroxyle ou alcanoyloxy en C₁-C₄; ou
- forme, conjointement avec R⁷ et les atomes qui les portent, des systèmes cycliques saturés ou partiellement insaturés, ayant de 3 à 12 chaînons formant le cycle; ou
- conjointement avec R⁸ et les atomes qui les portent, un système cyclique mono- ou bicyclique, saturé ou partiellement insaturé, comportant 5-12 chaînons formant le(s) cycle(s), qui peut contenir, en plus d'atomes de carbone, encore un atome de soufre qui peut éventuellement être oxydé en sulfoxyde ou sulfone; ou peut contenir un atome d'azote, le système cyclique pouvant éventuellement être substitué par le groupe amino;
R⁷ représente
- un atome d'hydrogène ou
- un groupe alkyle en C₁-C₆;
R⁸ représente
- un atome d'hydrogène,
- le groupe hydroxyle,
- un groupe alcanoyloxy en C₁-C₄ ou
- un groupe alkyle en C₁-C₈;
R⁹ et R¹⁰ représentent
- un atome d'hydrogène,
- un groupe alkyle en C₁-C₈ qui peut être substitué par un groupe
- amino,
- alkyl(C₁-C₄)amino,
- dialkyl(C₁-C₄)amino,
- mercapto,
- carboxyle,
- hydroxyle ou
- alcoxy en C₁-C₄,
- cycloalkyle en C₃-C₇,
- alcoxy(C₁-C₄) carbonyle,
- aryle en C₆-C₁₄, aryl (C₆-C₁₄)-alcoxy(C₁-C₄)-carbonyle, qui peuvent être substitués sur le fragment aryle comme décrit pour R⁴,
- Het ou
- Het-alkyle(C₁-C₄), Het étant défini comme décrit pour R⁴, ou
R⁹ et R¹⁰ forment ensemble, avec l'atome d'azote qui les porte, des systèmes cycliques monocycliques ou bicycliques, saturés, partiellement insaturés ou aromatiques, qui peuvent contenir en tant que chaînons formant le(s) cycle(s), en plus d'atomes de carbone, encore 1 ou 2 autres atomes d'azote, 1 atome de soufre ou 1 atome d'oxygène, et être substitués par des groupes alkyle en C₁-C₄, un ou plusieurs groupes amido (-CONH-) de la chaîne principale, dans les composés précédents de formule I, pouvant être remplacés par -CH₂NR¹¹-, -CH₂S-, -CH₂O-, -OCH₂-, -CH₂CH₂-, -CH=CH- (*cis* et *trans*), -COCH₂-, -CH(OH)CH_{2-,} -CH₂SO-, CH₂SO₂-, -COO-, -P(O)(OR¹²)CH₂- et -P(O) (OR¹²)NH-, ou également par un groupe amido à polarité inversée (-NHCO-);
groupes dans lesquels R¹¹ et R¹² représentent, indépendamment l'un de l'autre,
- un atome d'hydrogène ou
- un groupe alkyle en C₁-C₄;
ainsi que de leurs énantiomères et sels physiologiquement acceptables,
caractérisé en ce que l'on traite des α-aminoaldéhydes homochiraux de formule VII 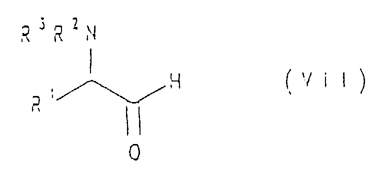 dans laquelle R¹, R² et R³ sont tels que définis plus haut, par un complexe de vanadium, obtenu in situ à partir de VCl₃, THF et de poudre de zinc, dans du THF et avec addition d'un complexant, en présence d'un contrôle simultané de tous les quatre centres de chiralité.

2. Procédé pour la préparation de composés de formule I selon la revendication 1, caractérisé en ce que
R¹ représente un reste de chaîne latérale des α-aminoacides 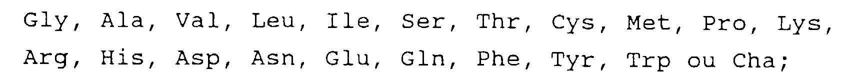
R² et R³ sont identiques ou différents et représentent
a) - un atome d'hydrogène,
b) - un radical de formule II dans lequel o et p = 0,
n = 0 ou 1 et
E représente l'un des α-aminoacides indiqués plus haut,
D représente R⁴ ou un radical de formule III ou IV, dans lequel R⁴ représente
b₁)
- un atome d'hydrogène,
- un groupe alkyle en C₁-C₉ qui est éventuellement une ou deux fois insaturé et qui est éventuellement substitué par jusqu'à 3 radicaux identiques ou différents, choisis parmi les radicaux
- hydroxyle,
- alcoxy en C₁-C₇,
- carbamoyle,
- alcanoyloxy en C₁-C₈,
- alcoxy(C₁-C₇)carbonyle,
- F, Cl,
- amino,
- alkyl(C₁-C₇)amino,
- dialkyl(C₁-C₇)amino,
- alcoxy(C₁-C₆) carbonylamino,
- aralcoxy(C₇-C₁₅)carbonyle,
- aralcoxy(C₇-C₁₅)carbonylamino,
- phénylalcoxy(C₁-C₄),
- 9-fluorénylméthoxycarbonylamino,
- alkyl (C₁-C₆)sulfonyle,
- alkyl(C₁-C₆)sulfinyle,
- alkyl(C₁-C₆)thio,
- aryle en C₆-C₁₄,
- aryl(C₆-C₁₄)-alkyle(C₁-C₆) ou
- aryloxy(C₆-C₁₄)-alkyle(C₁-C₆), le fragment aryle pouvant dans chaque cas être éventuellement substitué par 1, 2 ou 3 radicaux, identiques ou différents, choisis dans le groupe constitué par les substituants préférés du groupe alkyle en C₁-C₉, indiqués plus haut,
b₂)
- un radical de formule VI dans lequel
- R⁴a est défini comme R⁴ en b₁), et W représente -CO-, -O-CO-, -SO₂-, -SO-, -S-, -NHCO-, -CH(OH)-;
b₄)
- un groupe protecteur Fmoc, Z ou Boc de fonction amino,
R⁵ et R⁷ représentent des atomes d'hydrogène,
R⁶ est défini comme R⁴, et
R⁸ représente
- un atome d'hydrogène,
- le groupe hydroxyle,
- un groupe alcanoyloxy en C₁-C₄ ou
- un groupe alkyle en C₁-C₈.

3. Procédé pour la préparation de composés de formule I selon la revendication 1 ou 2, caractérisé en ce que l'un des radicaux R² et R³ représente un atome d'hydrogène.

4. Procédé pour la préparation de composés de formule I selon l'une des revendications 1 à 3, caractérisé en ce que les composés de formule I ont une configuration SRRS ou une configuration RSSR.

5. Procédé pour la préparation de composés de formule I selon l'une des revendications 1 à 4, caractérisé en ce que
R¹ représente un reste de chaîne latérale des α-aminoacides Ala, Val, Leu, Ile, Pro, Phe, Cha ou Tyr,
R² et R³ sont identiques ou différents et représentent
a) - un atome d'hydrogène,
b) - un radical de formule II dans lequel
o et p = 0,
n est 0 ou 1 et
E représente Ala, Val, Leu, Ile, Pro, Phe, Cha ou Tyr;
D représente R⁴ ou un radical de formule IV dans laquelle R⁴ représente
b₁)
- un atome d'hydrogène,
- un groupe alkyle en C₁-C₄,
- le groupe phényle ou naphtyle,
- phénylméthyle ou naphtylméthyle,
b₂)
- un radical de formule VI dans lequel R^{4a} est défini comme R⁴ en b₁₎ et W représente -CO-, -O-CO-, -SO₂-, -SO-, -S-, -NHCO-, -CH(OH)-, ou
b₄)
- un groupe protecteur Fmoc, Z ou Boc de fonction amino,
R⁵, R⁷ et R⁸ représentent des atomes d'hydrogène et
R⁶ est défini comme R⁴ en b₁).

6. Procédé pour la préparation de composés de formule I selon les revendications 1 à 5, caractérisé en ce que, dans un appareil purgé avec un gaz protecteur, on dispose au préalable du VCl₃ dans du THF, à des températures dans la plage allant de -78°C à la température d'ébullition, et on y ajoute successivement 0,5 à 1,0 équivalents de poudre de zinc et jusqu'à 9 équivalents d'un complexant et 0,2 à 1,0 équivalent d'un aldéhyde de formule VII, et on agite le mélange sous une atmosphère consistant en un gaz protecteur, à la température initiale respective, jusqu'à l'achèvement de la réaction.
